# EUROPEAN PATENT APPLICATION

(11) **EP 4 421 736 A1**
(43) Date of publication of application: **28.08.2024**
(21) Application number: 24158523.1
(22) Date of filing: 20.02.2024
(51) Int. Cl.: G06T 7/30

(54) **IMAGE PROCESSING APPARATUS, IMAGE PROCESSING METHOD, AND STORAGE MEDIUM**

(30) Priority: 22.02.2023 JP 2023026390; 08.08.2023 JP 2023129516
(71) Applicant: CANON KABUSHIKI KAISHA, Tokyo 146-8501 (JP)
(72) Inventor: MIYASA, Kazuhiro, Tokyo, 146-8501 (JP); TANAKA, Toru, Tokyo, 146-8501 (JP)
(74) Representative: TBK

(57) **Abstract**

An image processing apparatus includes: an obtaining unit configured to obtain at least one of first identification information and second identification information; a classification unit configured to classify, using the at least one piece of identification information, the plurality of first bones and the plurality of second bones into a first bone group including a bone that moves in association with a motion of a first part of the subject and a second bone group including a bone that moves in association with a motion of a second part different from the first part; and a registration unit configured to perform first registration between the plurality of first bones and the plurality of second bones classified into the first bone group and second registration between the plurality of first bones and the plurality of second bones classified into the second bone group.

## Description

### BACKGROUND OF THE INVENTION

### Field of the Invention

The present invention relates to an image processing apparatus, an image processing method, and a storage medium.

### Description of the Related Art

In the medical field, attempts have been made to visualize a temporal change of lesion or the like by presenting, to a user, a difference image generated from two images obtained by capturing images at different times by various modalities.

In Ryo Sakamoto, et al. "Temporal Subtraction of Serial CT Images with Large Deformation Diffeomorphic Metric Mapping in the Identification of Bone Metastases", Radiology, November 2017., there is disclosed a technique of performing registration with focus on bones between two three-dimensional images obtained by capturing images using a CT apparatus, thereby generating a difference image between the images. Also, patent literature 1 discloses a technique of recognizing the parts of a plurality of bones included in two three-dimensional images and performing registration processing for the images of the parts of bones associated with each other.

However, in the registration technique of disclosed in Ryo Sakamoto, et al. "Temporal Subtraction of Serial CT Images with Large Deformation Diffeomorphic Metric Mapping in the Identification of Bone Metastases", Radiology, November 2017., if the parts of bones whose motions are largely different or the parts of a plurality of bones with discontinuous deformation in the boundary portion are included in images to be registered, it may be difficult to correctly register the part of the bones.

On the other hand, in the registration technique of Japanese Patent Laid-Open No. 2017-63936, the parts of individual bones need to be recognized and associated. For this reason, for example, in a case where some bones are cut by surgery, misregistration may occur in registration, and it may be difficult to stably register the recognized bones.

In consideration of the above-described problems, the disclosed technique provides an image processing technique capable of correctly and stably performing registration between images.

### SUMMARY OF THE INVENTION

The present invention in its first aspect provides an image processing apparatus as specified in claims 1 to 25.

The present invention in its second aspect provides an image processing method as specified in claims 26 to 27.

The present invention in its third aspect provides a storage medium as specified in claim 28.

The present invention can solve to correctly and stably perform registration between images.

Further features of the present invention will become apparent from the following description of exemplary embodiments (with reference to the attached drawings).

### BRIEF DESCRIPTION OF THE DRAWINGS

Fig. 1 is a view showing the configuration of an image processing system according to the first embodiment;
Fig. 2 is a flowchart showing an overall processing procedure according to the first embodiment;
Fig. 3 is a view showing identification information of bones in a first medical image and a second medical image;
Fig. 4 is a view showing a plurality of groups of bones in the first medical image and the second medical image;
Fig. 5 is a view showing the configuration of an image processing system according to the second embodiment;
Fig. 6 is a flowchart showing an overall processing procedure according to the second embodiment;
Fig. 7 is a flowchart showing an overall processing procedure according to the third embodiment;
Fig. 8 is a view showing the configuration of an image processing system according to the fourth embodiment;
Fig. 9 is a flowchart showing an overall processing procedure according to the fourth embodiment;
Fig. 10 is a view showing the configuration of an image processing system according to the fifth embodiment; and
Fig. 11 is a flowchart showing an overall processing procedure according to the fifth embodiment.

### DESCRIPTION OF THE EMBODIMENTS

Hereinafter, embodiments will be described in detail with reference to the attached drawings. Note, the following embodiments are not intended to limit the scope of the claimed invention. Multiple features are described in the embodiments, but limitation is not made to an invention that requires all such features, and multiple such features may be combined as appropriate. Furthermore, in the attached drawings, the same reference numerals are given to the same or similar configurations, and redundant description thereof is omitted.

### <First Embodiment>

An image processing apparatus according to this embodiment is an apparatus that performs registration between two images captured at different times, thereby generating a difference image. More specifically, a plurality of bones are identified from two images, these are classified into a plurality of groups of bones that move in association with a plurality of different body parts of a subject, and then, registration between the images is performed for each group of bones.

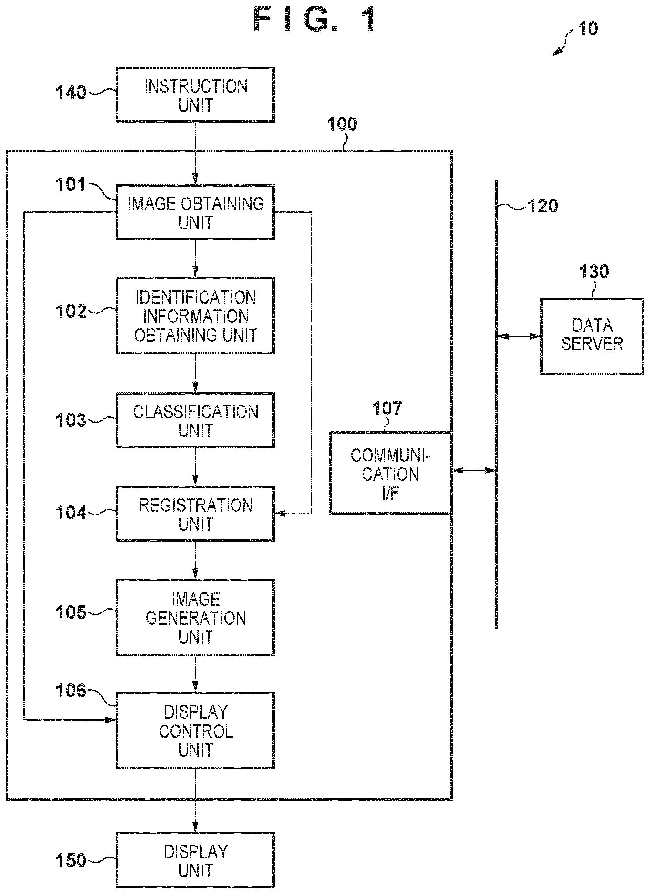

Fig. 1 is a view showing the configuration of an image processing system 10 according to the first embodiment. The image processing system 10 includes an image processing apparatus 100, and the image processing apparatus 100 includes a communication interface 107 (communication I/F) configured to be connected to a data server 130 via a communication network 120. The image processing apparatus 100 according to the first embodiment is an apparatus that performs registration between two images (a first medical image and a second medical image) captured at different times and generates a difference image between the first medical image and the second medical image based on the registration result.

The data server 130 holds a plurality of medical images. The data server 130 indicates, for example, a Picture Archiving and Communication Systems (PACS) that receives medical image data captured by a modality and stores and manages it via a network. In the following explanation, the data server 130 is assumed to hold, as the first medical image and the second medical image, a plurality of three-dimensional tomographic images obtained by capturing an image of a subject in advance under different conditions (different modalities, imaging modes, imaging dates/times, and body positions). In this embodiment, as an example of medical images, a description will be made assuming that the first medical image and the second medical image are three-dimensional tomographic images (three-dimensional medical images) obtained by performing imaging using an X-ray CT apparatus.

Note that in this specification, an axis representing the direction from the right to left of the subject will be defined as an X-axis, an axis representing the direction from the front to back of the subject will be defined as a Y-axis, and an axis representing the direction from the head to feet of the subject will be defined as a Z-axis. In addition, an XY cross-section will be defined as an axial plane, a YZ cross-section will be defined as a sagittal plane, and a ZX cross-section will be defined as a coronal plane. That is, the X-axis direction is a direction (to be referred to as a "sagittal direction" hereinafter) orthogonal to the sagittal plane. The Y-axis direction is a direction (to be referred to as a "coronal direction" hereinafter) orthogonal to the coronal plane. The Z-axis direction is a direction (to be referred to as an "axial direction" hereinafter) orthogonal to the axial plane. At this time, in a CT image that is a three-dimensional image formed as a set of two-dimensional tomographic images (slices), a slice plane of an image represents the axial plane, and a direction (to be referred to as a "slice direction" hereinafter) orthogonal to the slice plane represents the axial direction. Note that how to set a coordinate system is an example, and the coordinate system may be set by another definition.

The modality that captures a three-dimensional tomographic image may be an MRI apparatus, a three-dimensional ultrasonic imaging apparatus, a photoacoustic tomography apparatus, a PET/SPECT, an OCT apparatus, or the like. Furthermore, the first medical image and the second medical image may be any images if these are three-dimensional tomographic images as the target of registration. For example, these may be images captured in the same period by different modalities or in different imaging modes. Alternatively, these may be images obtained, for follow-up, by capturing an image of the same patient at the same body position using the same modality at different dates/times. Note that the first medical image and the second medical image are three-dimensional medical images (three-dimensional tomographic images) each formed as a set of two-dimensional tomographic images. The position and posture of each two-dimensional tomographic image are transformed into a reference coordinate system (a coordinate system in a space with respect to a subject as a reference) and held on the data server 130. At this time, the first medical image and the second medical image expressed on the reference coordinate system are input to the image processing apparatus 100 in accordance with an instruction of a user who operates an instruction unit 140. The instruction unit 140 includes various kinds of input devices that accept various kinds of instructions from the user. These are, for example, a mouse, a keyboard, a trackball, and a touch panel, and include various devices used by the user to input various kinds of information and processing requests.

The image processing apparatus 100 is an apparatus that performs image processing upon accepting a processing request of the user from the instruction unit 140 and outputs the result of the image processing to a display unit 150, and functions as a terminal apparatus for interpretation, which is operated by a user such as a doctor. More specifically, based on an instruction from the user via the instruction unit 140, a first medical image and a second medical image as the image processing target are obtained from the data server 130 as a pair of images (image pair) to be subjected to image processing. Then, the image processing apparatus 100 performs registration processing for the obtained first medical image and second medical image, generates a difference image between the first medical image and the second medical image based on the registration result, and outputs the difference image to the display unit 150.

The image processing apparatus 100 is formed by constituent elements to be described below. As for the following constituent elements, for example, one or a plurality of Central Processing Units (CPUs) functioning as the control unit of the image processing apparatus 100 execute programs, thereby constituting the functions of the units. The constituent elements of the image processing apparatus 100 may be formed as an integrated circuit or the like if the same functions can be obtained.

An image obtaining unit 101 obtains the information of a first medical image and a second medical image to be input to the image processing apparatus 100. An identification information obtaining unit 102 obtains identification information for identifying each of a plurality of bones from the input medical images. Based on the identification information of the plurality of bones obtained from the identification information obtaining unit 102, a classification unit 103 classifies the plurality of bones into a plurality of groups of bones associated with the motions of a plurality of different parts (body parts) of a subject. A registration unit 104 performs registration processing between the first medical image and the second medical image for each group of bones, and calculates a displacement field between the images for each group of bones. An image generation unit 105 generates, as a result image, a difference image between the first medical image and a deformed image of the second medical image based on a plurality of obtained displacement fields. A display control unit 106 performs display control for causing the display unit 150 to display the generated difference image or deformed image.

The display unit 150 is formed by an arbitrary device such as a Liquid Crystal Display (LCD), a Cathode Ray Tube (CRT), a plasma display, or an organic EL panel, and displays a medical image or the like for interpretation by a doctor. More specifically, the display unit 150 displays cross-section images of the first medical image and the second medical image, which are obtained from the image processing apparatus 100. The display unit 150 also displays a cross-section image of the deformed image and a cross-section image of the difference image, which are generated by the image processing apparatus 100.

Note that registration between images in this embodiment indicates processing of calculating deformation information for displacing each pixel position of one image to a corresponding pixel position of the other image. For example, if there are two images captured at different times, for each pixel position of one image serving as a reference, a corresponding pixel position of the other image is estimated, thereby calculating, as deformation information, a displacement field from the one image serving as the reference to the other image. Here, a displacement field between three-dimensional tomographic images is a three-dimensional vector field that stores displacements in the X, Y, and Z directions at each position in the images. One image (fixed image) serving as the reference of registration will be referred to as a reference image, and the other image (image to be deformed) will be referred to as a floating image. In this embodiment, the first medical image is handled as the reference image, and the second medical image is handled as the floating image.

### (Processing Procedure of Image Processing Apparatus 100)

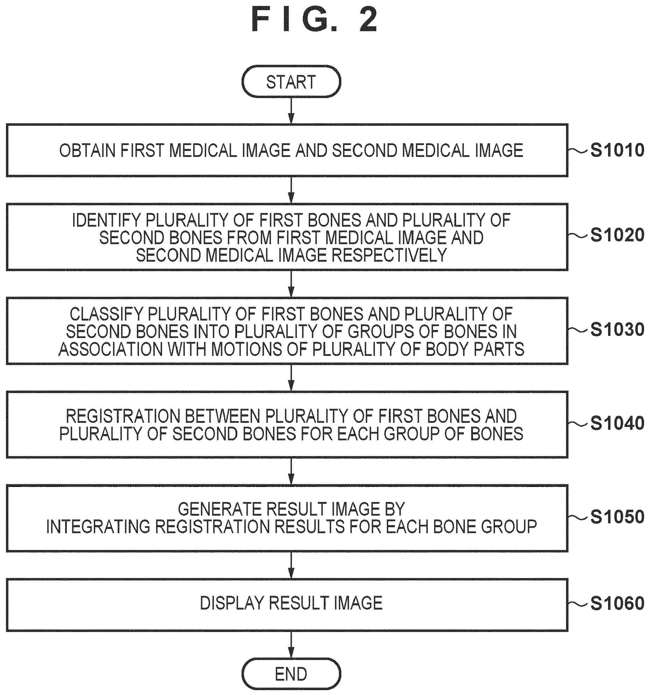

Fig. 2 is a flowchart showing an overall processing procedure performed by the image processing apparatus 100.

### (S1010: Obtain First Medical Image and Second Medical Image)

In step S 1010, the image obtaining unit 101 obtains, from the data server 130, a first medical image and a second medical image, which are designated by the user via the instruction unit 140. The image obtaining unit 101 then outputs the first medical image and the second medical image to the identification information obtaining unit 102, the registration unit 104, and the display control unit 106. Note that the image obtaining is not necessarily performed based on the user instruction, and may be performed by any other method. For example, when a captured image is saved in the data server 130, a pair of the image (first medical image) and a comparison target image (second medical image) may automatically be obtained based on a predetermined rule. As the comparison target image, a past image (for example, an image of the latest imaging date/time) of the same patient as the captured image is automatically obtained. As an example of automatic obtaining processing, for example, the first medical image can automatically be obtained as the latest inspection image of the inspection target subject, and the second medical image can automatically be obtained as the second newest inspection image of the same subject. Note that the method of automatic obtaining is not limited to this and, for example, the second medical image may automatically be obtained as the oldest inspection image of the same subject. Alternatively, only the first medical image may be designated by the user, and the second medical image may automatically be obtained based on a predetermined rule, as described above.

### (S1020: Identify Plurality of First and Second Bones from Each of Two Images)

In step S 1020, the identification information obtaining unit 102 identifies a plurality of bones from each of the first medical image and the second medical image. Here, a plurality of bones depicted in the first medical image will be referred to as a plurality of first bones, and a plurality of bones depicted in the second medical image will be referred to as a plurality of second bones. Also, information for identifying each of the plurality of first bones from the first medical image will be referred to as first identification information, and information for identifying each of the plurality of second bones from the second medical image will be referred to as second identification information.

The identification information obtaining unit 102 obtains at least one of the first identification information for identifying each of the plurality of first bones depicted in the first medical image obtained by capturing an image of the subject and the second identification information for identifying each of the plurality of second bones depicted in the second medical image different from the first medical image. The identification information obtaining unit 102 outputs at least one of the obtained first identification information and second identification information to the classification unit 103.

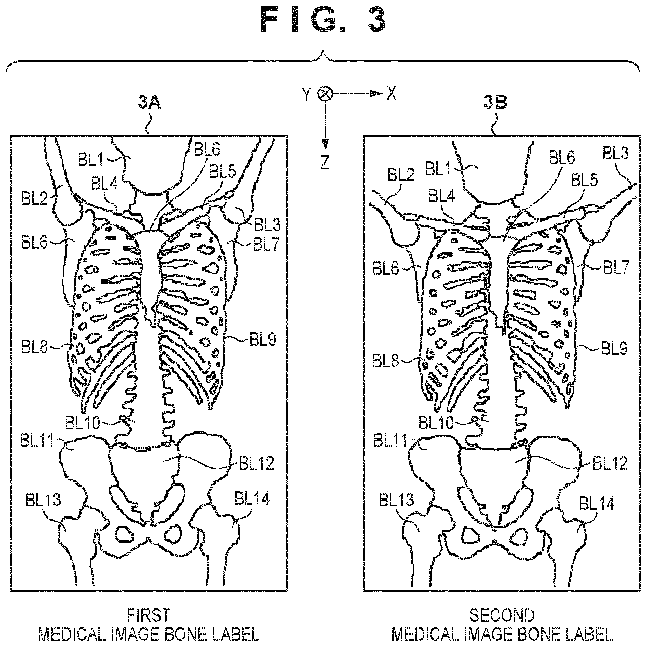

Fig. 3 is a view showing identification information of a plurality of bones in the first medical image and the second medical image. 3A of Fig. 3 shows the first identification information in the first medical image, and 3B of Fig. 3 shows the second identification information in the second medical image. In this embodiment, the identification information of a bone represents a label image having a label value that changes for each bone. Here, BL1 to BL14 in 3A and 3B of Fig. 3 indicate parts of bones.

More specifically, BL1 indicates a skull; BL2, a right upper arm bone; BL3, a left upper arm bone; BL4, a right collar bone; and BL5, a left collar bone. Also, BL6 indicates a right scapula bone; BL7, a left scapula bone; BL8, a right rib; an BL9, a left rib. BL10 indicates a backbone; BL11, an ilium; BL12, a sacrum; BL13, a right thigh bone; and BL14, left thigh bone. Label images with different label values assigned to the regions of the parts of these bones in the medical images are the identification information of the bones.

In this embodiment, the same identification information is set for bones belonging to the same part of the subject. For example, individual vertebrae in the backbone that is formed by a plurality of vertebrae belong to the same backbone, and the label value is assigned to the vertebrae in the same part, like the label BL10 indicating the backbone in 3A and 3B of Fig. 3.

Similarly, individual ribs on the right side of the subject (the left side in 3A and 3B of Fig. 3) are bones that form the right rib, and the label BL8 is assigned to the same part. Individual ribs on the left side of the subject (the right side in 3A and 3B of Fig. 3) are bones that form the left rib, and the label BL9 is assigned to the same part. Note the above-described classification is merely an example, and, for example, classification may be done not to the backbone but to different sections like cervical vertebra, thoracic vertebra, and lumbar vertebra. Additionally, bones belonging to the same part need not always be assigned the same label, and bones may be identified in a unit that can substantially be regarded as a rigid body such that, for example, a different label is assigned to each vertebra.

Here, a known method can be used to identify bones in an image. For example, bones may be identified by a region extraction method using known machine learning. In this case, a learned inference model that has learned the label regions of bones of many cases in advance may be constructed, and the label region of a bone may be extracted by inputting a target medical image to the inference model.

As the region extraction method using known machine learning, for example, a method such as a Convolutional Neural Network (CNN) can be used. Note that the region extraction method using machine learning is not limited to the Convolutional Neural Network (CNN) and may be another known technique. For example, after a whole bone region is extracted from a target medical image by a known extraction method, the statistical shape model of bones having identification information for each bone part is applied to the bone region extracted from the medical image. There are also a Deep Neural Network (DNN), a Recurrent Neural Network (RNN), a Generative Adversarial Network (GAN), and the like, and any of the methods is applicable.

The identification information of bones may be obtained by any other method. For example, the identification information obtaining unit 102 may be configured to obtain the identification information of bones associated with the first medical image and the second medical image in advance and saved in the data server 130 without performing bone identification processing.

### (S1030: Classify Plurality of First Bones and Plurality of Second Bones into Plurality of Groups of Bones)

In step S 1030, based on the first identification information and the second identification information, the classification unit 103 classifies the plurality of first bones and the plurality of second bones, for each of body parts that make different motions, into a plurality of groups of bones that move in association with the motions. Based on at least one of the first identification information and the second identification information, the classification unit 103 classifies the plurality of first bones and the plurality of second bones into a first bone group including bones that move in association with the motion of a first part (body part) of the subject and a second bone group including bones that move in association with the motion of a second part (body part) different from the first part (body part). Also, using at least one of the pieces of identification information, the classification unit 103 classifies the plurality of first bones and the plurality of second bones into a third bone group including bones that move in association with the motion of a third part (body part) different from the first part (body part) and the second part (body part). Then, the classification unit 103 outputs the information of the plurality of classified groups of bones to the registration unit 104. For example, the first part is the trunk of the subject, the second part is one of the limbs of the subject, and the third part is one of the limbs of the subject different from the second part. The second part and the third part physically continue to the first part, and the second part and the third part are parts that can move independently of each other.

Here, body parts that make different motions are body parts having such a relationship that the manner to move the body parts or the manner to rotate is different when the subject moves the body. For example, like the relationship between the trunk and the limbs, the motion of the trunk is a motion obtained mainly by bending the backbone to back and forth and right and left, and the motion of the limbs is a motion to rotate arms or legs in various directions with respect to the connection position to the trunk as an axis. Here, these are defined as body parts that make different motions because the manner to move the body parts or the manner to rotate is different.

On the other hand, body parts that make the same motion are body parts having such a relationship that the manner to move the body parts or the manner to rotate is the same when the subject moves the body. For example, like the relationship between the chest and the abdomen, body parts whose motions belong to a motion obtained by bending the backbone to back and forth and right and left, that is, the chest and the abdomen are defined as body parts that make the same motion.

In addition, a bone that moves in association with the motion of a body part indicates a bone that moves together with the motion of the body part when moving the body part. For example, a bone that moves in association with the motion of the right arm in the limbs is mainly a bone of the arm or a scapula bone. Here, like the scapula bone defined as a bone that moves in association with the motion of the arm, a bone that is included in a certain body part but its motion is mainly associated with the motion of another body part is defined as a bone that moves in association with the motion of the other body part.

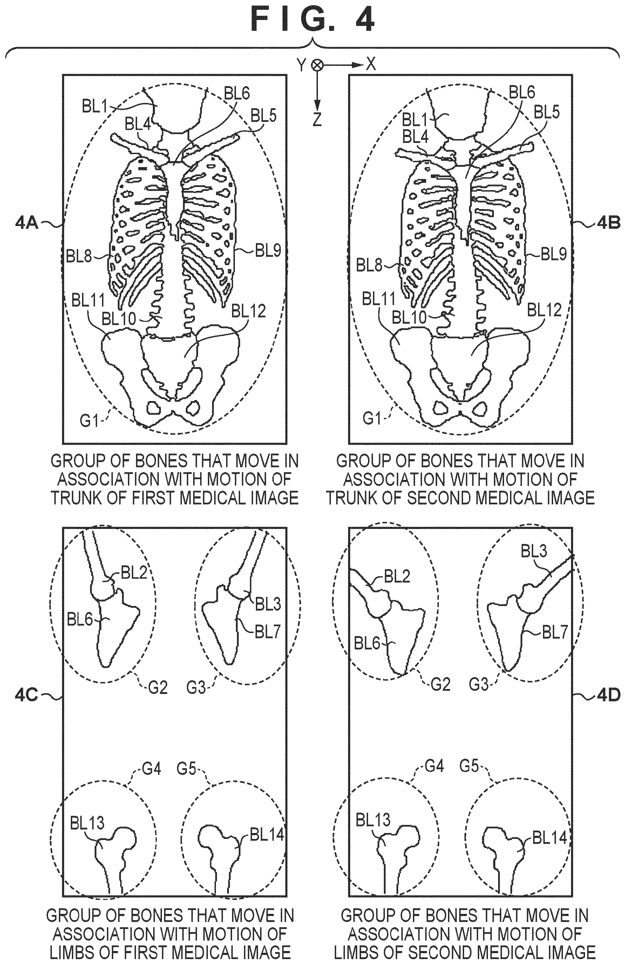

Fig. 4 is a view showing the groups of the plurality of first bones in the first medical image and the groups of the plurality of second bones in the second medical image. 4A of Fig. 4 shows a group of bones that move in association with the motion of the trunk in the first medical image. 4B of Fig. 4 shows a group of bones that move in association with the motion of the trunk in the second medical image. 4C of Fig. 4 shows four groups of bones that move in association with the motions of the limbs in the first medical image. 4D of Fig. 4 shows four groups of bones that move in association with the motions of the limbs in the second medical image. BL1 to BL14 in 4A to 4D of Fig. 4 indicate the parts of bones, like 3A and 3B of Fig. 3.

In this embodiment, the plurality of first bones and the plurality of second bones are classified into a group G1 (for example, a first bone group) of bones that move in association with the motion of the trunk and groups G2, G3, G4, and G5 (for example, second bone groups) of bones that move in association with the motions of the limbs, which are different body parts. However, the method of classifying the plurality of bones is not limited to this, and another classification result may be obtained. For example, the right collar bone BL4 may be classified into the group G2 of bones of the right arm, and the left collar bone BL5 may be classified into the group G3 of bones of the left arm.

At this time, as the result of classification, the classification unit 103 employs a group of bones that commonly exist in the first medical image and the second medical image as the final classification result, and does not employ a group of bones that exist only in one image as the final classification result. Using at least one of the first identification information concerning each of the plurality of first bones and the second identification information concerning each of the plurality of second bones depicted in the second medical image, the classification unit 103 identifies bones that are commonly depicted between the first medical image and the second medical image. The classification unit 103 then employs, as the final group of bones (the first bone group and the second bone group), a common group of bones in which the commonly depicted bones exist. The classification unit 103 classifies the plurality of first bones into the first bone group, and classifies the plurality of second bones into the second bone groups.

In the example shown in 4A to 4D of Fig. 4, to the bone group G1, the skull BL1, the right collar bone BL4, the left collar bone BL5, the breast bone BL6, the right rib BL8, the left rib BL9, the backbone BL10, the ilium BL11, and the sacrum BL12, which move in association with the motion of the trunk, are classified.

To the bone group G2, the right upper arm bone BL2 and the right scapula bone BL6, which move in association with the motion of the right arm, are classified. To the bone group G3, the left upper arm bone BL3 and the left scapula bone BL7, which move in association with the motion of the left arm, are classified. To the bone group G4, the right thigh bone BL13 that moves in association with the motion of the right leg is classified. To the bone group G5, the left thigh bone BL14 that moves in association with the motion of the left leg is classified. Hence, the whole trunk region including a part of the head and a part of each leg is common to the first medical image and the second medical image. That is, since all the bone groups G1 to G5 are common, the classification unit 103 directly employs the bone groups G1 to G5 as the final classification result.

On the other hand, for example, in a case where the imaging range of the first medical image is the whole trunk region, as described above, and the imaging range of the second medical image includes only the upper half body, the five groups G1 to G5 exist in the plurality of first bones in the first medical image. However, in the second medical image, only the three groups G1, G2, and G3 exist. That is, since only the three groups G1, G2, and G3 are common, the classification unit 103 employs the three groups G1, G2, and G3 as the final classification result, but the groups G4 and G5, which exist only in the plurality of first bones, are not employed as the final classification result.

Also, in a case where the imaging range of the first medical image is the whole trunk region, and the imaging range of the second medical image includes only the lower half body, the five groups G1 to G5 exist in the plurality of first bones in the first medical image. However, in the second medical image, only the three groups G1, G4, and G5 exist. That is, since only the three groups G1, G4, and G5 are common to the plurality of first bones and the plurality of second bones, the classification unit 103 employs the three groups G1, G4, and G5 as the final classification result, but the groups G2 and G3, which exist only in the plurality of first bones, are not employed as the final classification result.

Hence, the common groups in which the bones commonly depicted between the first medical image and the second medical image exist change depending on the degree of overlap of the imaging range between the first medical image and the second medical image. As described above, in this embodiment, since the classification is performed for, in the plurality of bones identified in step S1030, only for the groups common to the first medical image and the second medical image, not all the bones depicted in each medical image are classified.

However, the bone group classification method according to this embodiment is not limited to the above-described classification method. For example, after the bones of the same label are associated in advance between the plurality of first bones and the plurality of second bones, only a plurality of pairs of bones associated may be classified into each group of bones. In this case, for example, in the above-described case where the imaging range of the first medical image is the whole trunk region, and the imaging range of the second medical image includes only the upper half body, the following difference occurs.

That is, in the above-described case of association on a group basis, to the group G1 of bones associated with the motion of the trunk, the bones BL1, BL4, BL5, BL6, BL8, BL9, BL10, BL11, and BL12 are classified in the first medical image.

On the second medical image, since the ilium BL 11 and the sacrum BL12 are not included in the upper half body, only the bones BL1, BL4, BL5, BL6, BL8, BL9, and BL10 are classified.

On the other hand, in a case where the bones of the same label are associated, only the bones BL1, BL4, BL5, BL6, BL8, BL9, and BL10, which are common to the first medical image and the second medical image, are classified to the bone group G1.

Note that in this embodiment, an example in which the plurality of first bones and the plurality of second bones are classified into the first bone group associated with the motion of the trunk and the second to fifth bone groups associated with the motions of the limbs has been described. However, the present invention is not limited to this. For example, if the motion difference between the trunk and the limbs is small between the plurality of medical images, the bones associated with the motions of the limbs and the bones associated with the motion of the trunk may not be distinguished.

For example, if the group G1 of bones associated with the motion of the trunk is substantially registered by a known method between the plurality of medical images, the positions of the images are registered with respect to the group G1 as the reference. After that, if the difference in position/orientation is not so large between the plurality of medical images in one of the groups G2 to G5, the group may be included in the group G1.

At this time, as the method of substantially registering the group G1, after the surface point groups of bones included in each group are sampled, rigid body registration is performed by a method such as Iterative Closest Points (ICP). To calculate the difference in position/orientation between the images in the groups G2 to G5, the average distance between the surface point groups of the bones included in each group can be employed between the plurality of medical images. This makes it possible to avoid classifying bones to different groups although the motion changes little between the trunk and the limbs between the plurality of medical images.

### (S1040: Perform Registration between Plurality of First Bones and Plurality of Second Bones for Each Group of Bones)

In step S1040, the registration unit 104 performs registration between the plurality of first bones and the plurality of second bones for each group of bones, and outputs a generated displacement field to the image generation unit 105.

In this embodiment, the registration unit 104 cuts out the image of a bone region belonging to each group of bones from each of the first medical image and the second medical image based on the information of the plurality of groups of bones obtained from the classification unit 103. More specifically, letting I1 be the first medical image and I2 be the second medical image, the registration unit 104 obtains (cuts out), from the first medical image I1 and the second medical image I2, images I1_{G1} and I2_{G1} of bone regions belonging to the group G1 of bones associated with the motion of the trunk. In addition, the registration unit 104 obtains (cuts out), from the first medical image I1 and the second medical image 12, images I1_{G2} to I1_{G5} and I2_{G2} to I2_{G5} of bone regions belonging to the groups G2 to G5 of bones associated with the motions of the limbs.

At this time, for the image I1_{G1}, the bone region belonging to the bone group G1 need not always be cut out from the first medical image. For example, an image obtained by replacing pixel values in the bone regions belonging to the groups G2 to G5 of bones associated with the motions of the limbs with a predetermined pixel value (for example, a pixel value close to a peripheral tissue outside the bones) may be obtained as the image I1_{G1} from the first medical image. Note that the image I2_{G1} can be obtained by the same method.

The registration unit 104 then performs registration, for each group, between the images I1_{G1} to I1_{G5} cut out from the first medical image I1 and the images I2_{G1} to I2_{G5} cut out from the second medical image 12. The registration unit 104 deforms the images I2_{G1} to I2_{G5} that are floating images in this embodiment, thereby generating displacement fields D_{G1} to D_{G5} used to make the images substantially match the images I1_{G1} to I1_{G5} that are reference images.

For example, in registration between the image I1_{G1} and the image I2_{G1}, the registration unit 104 obtains, as the first displacement field D_{G1}, the displacement amount between a position in a region corresponding to the first bone group in the first medical image and a position in a region corresponding to the first bone group in the second medical image. The registration unit 104 then performs first registration based on the first displacement field D_{G1} that is used to cause the image I1_{G1} of the bone region belonging to the first bone group in the first medical image match the image I2_{G1} of the bone region belonging to the first bone group in the second medical image.

Also, in registration between the images I1_{G2} to I1_{G5} and the images I2_{G2} to I2_{G5}, the registration unit 104 obtains, as the second displacement fields D_{G2} to D_{G5}, the displacement amounts between positions in regions corresponding to the second bone groups in the first medical image and positions in regions corresponding to the second bone groups in the second medical image. The registration unit 104 then performs second registration based on the second displacement fields D_{G2} to D_{G5} that are used to cause the images I1_{G2} to I1_{G5} of the bone regions belonging to the second bone groups in the first medical image match the images I2_{G2} to I2_{G5} of the bone regions belonging to the second bone groups in the second medical image.

Note that the registration unit 104 can perform registration by a known image processing method for uniformly evaluating an entire image. For example, registration can also be performed by obtaining a displacement field by deforming one image such that the image similarity between the plurality of medical images becomes high.

As the image similarity, Sum of Squared Difference (SSD) that is generally used or a known method such as a mutual information amount or a cross-correlation coefficient can be used. As an image deformation model, a known deformation model such as affine transformation, Free Form Deformation (FFD), a Demons algorithm, or Large Deformation Diffeomorphic Metric Mapping (LDDMM) can be used.

As described above, when independent registration is performed for each of the groups of bones associated with the motions of body parts that make different motions, the entire registration can be decomposed to registration between groups of bones that make similar motions. In addition, it is not necessary to directly handle discontinuous deformation that may occur in the boundary portion between the groups of bones.

More specifically, a plurality of bones are separated and classified, as the group G2 of bones of the right arm, from the group G1 of bones of the trunk to which the bones are originally physically connected, thereby performing registration with focus on the right arm that makes a motion different from the trunk and preventing registration accuracy from lowering.

Also, when a rib belonging to the group G1 of bones of the trunk and a scapula bone belonging to the group G2 of bones of the right arm are independently registered, it is not necessary to directly handle discontinuous deformation that may occur between these, and lowering of registration accuracy caused by this can be prevented.

That is, in a case where the motions of a plurality of bones belonging to different body parts are largely different between a plurality of medical images, or discontinuous deformation occurs in the boundary portion, registration accuracy can be prevented from lowering.

Furthermore, a plurality of bones are separated and classified, as the group G3 of bones of the left arm, from the group G1 of bones of the trunk to which the bones are originally physically connected, thereby performing registration with focus on the left arm that makes a motion different from the trunk. It is possible to focus on each of the right arm and the left arm, which can operate independently of each other with respect to the trunk and perform registration independently of each other.

Also, registration is performed by associating the bones on a bone group basis between the plurality of medical images. Hence, even if an abnormal part exists in a part of one group of bones, the plurality of first bones and the plurality of second bones are registered such that all a plurality of bones included in the group match between the plurality of medical images. Since registration is performed such that bones in a normal structure around the abnormal part match, it is possible to stably perform registration even if an abnormal part exists.

More specifically, even if some vertebrae of the backbone in the second medical image are crushed due to a fracture or the like, registration is performed such that all the plurality of bones associated with the motion of the trunk including the backbone match between the plurality of medical images. Hence, registration is performed such that normal vertebrae or ribs around the vertebrae crushed due to the fracture match those in the first medical image. This makes it possible to suppress lowering of accuracy in entire registration due to the influence of a local difference that occurs in each bone between the first medical image and the second medical image.

### (S1050: Integrate Registration Results to Generate Result Image)

In step S 1050, the image generation unit 105 generates an image (result image) in which registration results are integrated based on the displacement fields obtained by the process of step S 1040. That is, based on the displacement fields obtained by the registration unit 104, the image generation unit 105 generates a deformed image by deforming the second medical image such that it matches the first medical image. Also, the image generation unit 105 generates a difference image by subtracting the deformed image from the first medical image. The image generation unit 105 then outputs the generated deformed image and difference image as registration results to the display control unit 106.

In this embodiment, the image generation unit 105 generates an integrated displacement field Dₜₒₜₐₗ by integrating the plurality of obtained displacement fields D_{G1} to D_{G5}. A more detailed method will be described below. The plurality of displacement fields D_{G1} to D_{G5} store displacement amounts from positions in the regions corresponding to the bone groups G1 to G5 in the first medical image serving as a reference to positions in the regions of the corresponding bone groups G1 to G5 in the second medical image. Hence, each of the plurality of displacement fields D_{G1} to D_{G5} stores a displacement amount corresponding to each position in a different region using the first medical image as the reference. Also, since the plurality of displacement fields D_{G1} to D_{G5} are data that store only partial displacement amounts, these need to be integrated to displace the position of the whole region of the plurality of bones in the image.

Hence, the image generation unit 105 first sets a region of a blank displacement field which has the same size as the first medical image and in which all displacement amounts at positions are 0. Then, the image generation unit 105 stores the displacement amounts of the plurality of displacement fields D_{G1} to D_{G5} in the regions of the corresponding groups G1 to G5 on the blank displacement field, thereby generating the integrated displacement field Dₜₒₜₐₗ in which the displacement amounts of the different displacement fields are stored at different positions.

For example, the image generation unit 105 stores the displacement amount of the first displacement field D_{G1} in the region of the first bone group in the set region, and stores the displacement amounts of the second displacement fields D_{G2} to D_{G5} in the regions of the second bone groups in the set region. The image generation unit 105 then generates the integrated displacement field Dₜₒₜₐₗ in which the displacement amounts of the different displacement fields D_{G1} to D_{G5} are stored at different positions in the set region.

Using the integrated displacement field Dₜₒₜₐₗ, the image generation unit 105 generates a deformed image by deforming the second medical image, and generates a difference image by subtracting the deformed image from the first medical image. Note that if the purpose is to output the difference image (the deformed image is not needed), the deformed image need not always be generated as an image. For example, the pixel information (for example, the pixel value) of each pixel of the deformed image may be calculated using the integrated displacement field (the deformed image is not saved as an image), and the difference value between the pixel information of a corresponding pixel of the first medical image and the pixel information of each pixel of the deformed image may be obtained, thereby generating only the difference image.

As another method, using the plurality of obtained displacement fields D_{G1} to D_{G5}, the image generation unit 105 generates deformed images DI2_{G1} to DI2_{G5} by deforming the medical images I2_{G1} to I2_{G5} cut out from the second medical image and thus generated in step S1040. The deformed images DI2_{G1} to DI2_{G5} are images that substantially match the medical images I1_{G1} to I1_{G5} cut out from the first medical image. For this reason, the deformed images DI2_{G1} to DI2_{G5} store, at positions in the regions corresponding to the bone groups G1 to G5 in the first medical image, pixel information (for example, pixel values) at positions in corresponding regions in the second medical image using the first medical image as the reference.

Since the deformed images DI2_{G1} to DI2_{G5} are data that store only partial pixel information, these need to be integrated to generate the deformed image of the whole region of the plurality of bones in the image. Hence, the image generation unit 105 first generates a blank image which has the same size as the first medical image and in which all the values of pixels are set to 0. Then, the image generation unit 105 stores the pixel information of the deformed images DI2_{G1} to DI2_{G5} in the blank image, thereby generating an integrated deformed image DI2ₜₒₜₐₗ in which the pieces of pixel information are stored at different positions. The image generation unit 105 subtracts the integrated deformed image DI2ₜₒₜₐₗ from the first medical image I1, thereby generating a difference image.

As still another method, the image generation unit 105 generates the deformed images DI2_{G1} to DI2_{G5} using the plurality of obtained displacement fields D_{G1} to D_{G5}, as described above, and then generates difference images SI_{G1} to SI_{G5} by subtracting the deformed images DI2_{G1} to DI2_{G5} from the medical images I1_{G1} to I1_{G5} cut out from the first medical image. Regions where the difference values of the difference images SI_{G1} to SI_{G5} are stored correspond to the medical images I1_{G1} to I1_{G5}, respectively. For this reason, the difference images SI_{G1} to SI_{G5} store, at positions in the regions corresponding to the bone groups G1 to G5 in the first medical image, the difference values at positions in corresponding regions in the second medical image using the first medical image as the reference. Since the difference images SI_{G1} to SI_{G5} are data that store only partial difference values, these need to be integrated to generate the difference image of the whole region of the plurality of bones in the image. Hence, the image generation unit 105 first generates a blank image which has the same size as the first medical image and in which all the values of pixels are set to 0. Then, the image generation unit 105 stores the difference values of the difference images SI_{G1} to SI_{G5} in the blank image, thereby generating an integrated difference image SI in which the difference values are stored at different positions.

As yet another method, the image generation unit 105 generates a deformed image DI2₁ by deforming the second medical image using the displacement amount of the first displacement field D_{G1} and deformed images DI2₂ to DI2₅ by deforming the second medical image using the second displacement fields D_{G2} to D_{G5}. The image generation unit 105 generates difference images SI₁ to SI₅ by subtracting the deformed image DI2₁ and the deformed images DI2₂ to DI2₅ from the first medical image. The difference images SI₁ to SI₅ store, not only in the bone region of each group of bones but also at all positions in the first medical image, the difference values for corresponding positions in the second medical image as the results of registration.

The image generation unit 105 first generates a blank image which has the same size as the first medical image and in which all the values of pixels are set to 0. Then, the image generation unit 105 stores the difference values on the difference images of target bone groups in the difference images SI₁ to SI₅ at positions in regions of the blank image corresponding to the regions of the groups G1 to G5 of the bones in the first medical image, thereby generating the integrated difference image SI. For example, at a position in a region of the blank image corresponding to the bone group G2 in the first medical image, the image generation unit 105 stores the difference value of the difference image SI₂ generated based on the displacement field D_{G2} for the bone group G2 as the target.

Also, in any one of the difference images SI₁ to SI₅, the difference values may be stored at all other positions in the blank image, which do not correspond to the regions of the groups of bones, to generate the integrated difference image SI. For example, the difference values of the difference images SI₂ to SI₅ may be stored at positions in regions of the blank image corresponding to the bone groups G2 to G5 in the first medical image, and the difference value of the difference image SI₁ may be stored at all positions in the blank image other than the regions corresponding to the bone groups G2 to G5 in the first medical image, thereby generating the integrated difference image SI.

That is, the difference value of one of the difference images SI₁ to SI₅ is stored as the difference value at each position in the integrated difference image SI. According to this, even if there exists a bone region that is not classified into any of the bone groups G1 to G5 because of insufficient bone region extraction, difference values can be stored in all bone regions in the integrated difference image SI without any omission.

However, if the difference value of one of the difference images SI₁ to SI₅ is stored at each position in the integrated difference image SI, mismatching in the registration result for each group of bones may occur near the boundary portion between the groups of bones in the integrated difference image SI. For example, in a region other than the bone region between the right thigh bone and the ilium in the first medical image, a part of the region of the right thigh bone that is not included in the bone group G1 in the deformed image DI2₁ and a part of the region of the ilium that is not included in the bone group G4 in the deformed image DI2₄ may overlap on the image space. In this overlap region, each of the difference images SI₁ and SI₄ generated from the deformed images is a difference between a bone region and a region other than the bone region in the first medical image. For this reason, if one of the difference values is stored as the difference value of the integrated difference image SI, a difference value may be generated between the right thigh bone and the ilium regardless of which difference value is stored.

Even if regions overlap on the image space, in this embodiment, regions that do not belong to the bone groups G1 to G5 are set as invalid regions in the deformed images DI2₁ to DI2₅. If the invalid regions of a plurality of deformed images overlap, the registration result is determined as invalid, and a predetermined difference value (for example, 0) is stored in the blank image, thereby generating the integrated difference image SI. This can eliminate mismatching in the registration result, which may occur in the integrated difference image SI.

In this embodiment, the image generation unit 105 saves the generated result images (the deformed image and the difference image) in a storage unit (not shown). Hence, if the result images should be obtained again after the end of the processing of the image processing apparatus 100, the image generation unit 105 can easily obtain the result images by loading the result images saved in the storage unit. However, the generated result images need not always be saved in the storage unit (not shown).

In addition, both the deformed image of the second medical image and the difference image need not always be generated as the result images. Only one of the deformed image of the second medical image and the difference image may be generated. Alternatively, without generating the deformed image and the difference image, an integrated displacement field may be saved in the storage unit (not shown) as information for generating these. Generation of the deformed image and the difference image from the integrated displacement field may be performed by another apparatus.

### (S1060: Display Image)

In step S1060, the display control unit 106 performs control of displaying, on the display unit 150, the cross-section images of the result images obtained from the image generation unit 105. The display control unit 106 also performs control of displaying the cross-section images of the first medical image and the second medical image on the display unit 150. The display unit 150 is formed by an arbitrary device such as an LCD or a CRT, and displays a medical image or the like for interpretation by a doctor. A GUI configured to obtain an instruction from the user such as a doctor is arranged on the display unit 150. The user can freely switch the first medical image and the second medical image, which are being displayed, to the difference image or the deformed image using the GUI. Also, the display control unit 106 can control to combine images and display these on the display unit 150 based on an instruction from the GUI.

Note that if the result images are saved or output to the outside, the display of the result images is not always necessary. Also, if the purpose is to perform image processing using the result images, save and display of the result images need not be performed. For example, a portion where pixel information (that is a pixel value) in the difference image is large, that is, a portion where the change of pixel information between the medical images is large may be detected and output as an abnormal region candidate, or the result images (the deformed image and the difference image) may be input to a discriminator to discriminate the presence/absence of an abnormality. The processing of the image processing apparatus 100 is thus executed.

According to this embodiment, by performing registration independently for each of the groups of bones associated with the motions of body parts that make different motions, the following effects can be obtained. That is, in a case where the motions of a plurality of bones belonging to different body parts between images are largely different, or discontinuous deformation occurs in the boundary portion, lowering of registration accuracy can be suppressed. Also, since registration is performed by associating bones on a bone group basis between images, even if an abnormal part exists in a part of one group of bones, registration can stably be performed. That is, according to this embodiment, it is possible to correctly and stably perform registration between images.

### (Modification 1)

In the first embodiment, a configuration has been described in which after the plurality of bones are identified from each of the first medical image and the second medical image in step S1020, the bones are classified into a plurality of groups of bones in step S1030.

However, identification of the plurality of bones and classification of the plurality of bones need not always be performed by this method. For example, in step S1020, the bones may be identified in the same unit as the groups of bones to be classified in step S1030. More specifically, the identification information obtaining unit 102 may identify the bones BL1, BL4, BL5, BL8, BL9, BL10, BL11, and BL12 included in the bone group G1 shown in 4A and 4B of Fig. 4 as identification information of the same label value. Also, the identification information obtaining unit 102 may identify the bones BL2 and BL6 included in the bone group G2, the bones BL3 and BL7 included in the bone group G3, the bone BL13 included in the bone group G4, and the bone BL14 included in the bone group G5 as identification information of the same label value. When the bones are identified first for each of groups of bones associated with the motions of body parts that make different motions, the classification processing in step S1030 can be skipped.

### <Second Embodiment>

In the first embodiment, a configuration in which a plurality of bones are identified from a first medical image and a second medical image, the bones are classified into a plurality of groups of bones, and registration is performed for each group of bones has been described.

In the second embodiment, a configuration in which a plurality of bones are identified from one of a first medical image and a second medical image, and the bones are classified into a plurality of groups of bones will be described.

An identification information obtaining unit 102 obtains identification information of a plurality of bones identified from one of a first medical image and a second medical image. Based on the identification information, a classification unit 103 classifies the plurality of bones identified from one of the images into groups of bones that move in association with different motions for each of parts of a subject, which make different motions.

A registration unit 104 performs initial registration of one medical image to the other medical image, and after that, associates the plurality of groups of bones in one medical image with bone regions in the other medical image, thereby obtaining the information of the plurality of groups of bones in the plurality of medical images. Finally, registration is performed for each of the plurality of groups of bones associated in both images.

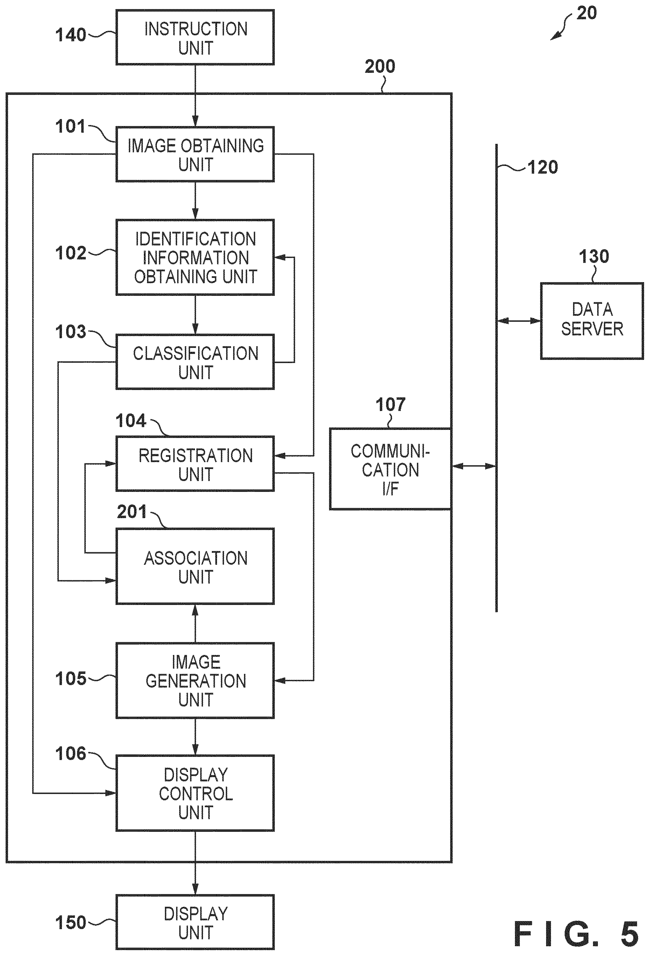

Fig. 5 is a view showing the configuration of an image processing system 20 according to the second embodiment. An image processing apparatus 200 according to the second embodiment includes an association unit 201. The configuration other than the association unit 201 is the same as in Fig. 1, and a description thereof will be omitted. The association unit 201 performs processing of associating information of a plurality of groups of bones set in one of two input images, that is, a first medical image and a second medical image with the other image.

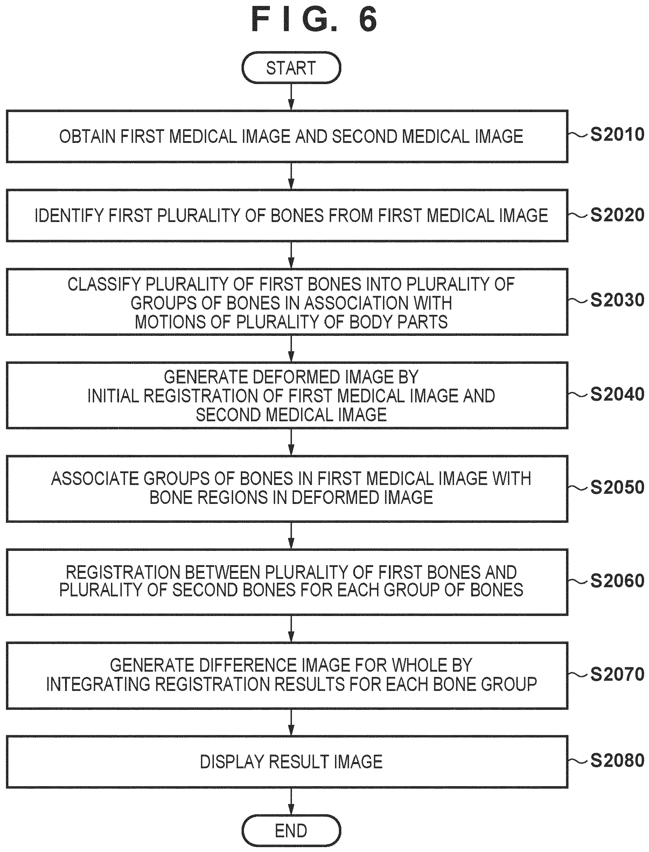

Processing according to this embodiment will be described below with reference to the flowchart of Fig. 6. Note that steps S2010 and S2060 to S2080 in the flowchart showing the overall processing procedure performed by the image processing apparatus 200 are the same as steps S1010 and S1040 to S1060 in the first embodiment, and a description thereof will be omitted.

Only differences from the first embodiment will be described below with reference to the flowchart of Fig. 6.

### (S2020: Identify Plurality of Bones Only from First Medical Image)

In step S2020, the identification information obtaining unit 102 identifies a plurality of bones from one of a first medical image and a second medical image. The identification information obtaining unit 102 then outputs the information of the plurality of identified bones as identification information to the classification unit 103. In this embodiment, for example, the identification information obtaining unit 102 identifies only a plurality of first bones depicted in the first medical image, and obtains only first identification information that is the identification information thereof. The processing of identifying a plurality of bones from one medical image is the same as in step S1020, and a description thereof will be omitted.

### (S2030: Classify Only Plurality of First Bones into Plurality of Groups of Bones)

In step S2030, based on the identification information, the classification unit 103 classifies, for each of body parts of the subject, that make different motions, the plurality of bones identified from the medical image out of the first medical image and the second medical image, which has undergone the processing in step S2020, into a plurality of groups of bones that move in association with the motions.

Then, the pieces of information of the plurality of classified groups of bones are output to the association unit 201. In this embodiment, the plurality of first bones are classified into a plurality of groups of bones based on the first identification information. The processing of classifying the plurality of bones into the plurality of groups of bones is the same as in step S1030, and a description thereof will be omitted.

### (S2040: Perform Initial Registration between First Medical Image and Second Medical Image)

In step S2040, the registration unit 104 performs initial registration between the first medical image and the second medical image, and generates a displacement field for deforming the second medical image to the first medical image. The registration unit 104 then outputs the generated displacement field to an image generation unit 105. Next, the image generation unit 105 generates a deformed image by deforming the second medical image based on the displacement field obtained from the registration unit 104. The image generation unit 105 then outputs the generated deformed image to the association unit 201.

Here, the initial registration can be performed by a known image processing method of uniformly evaluating an entire image, as in step S 1040. In this embodiment, however, since bone group information does not exist in the second medical image, unlike step S1040, the registration unit 104 does not generate an image in which a bone region corresponding to each group of bones is cut out. For this reason, at the time of registration, the registration unit 104 performs processing of, for example, extracting bone regions from the first medical image and the second medical image in advance and performs registration for making the bone regions substantially match between the first medical image and the second medical image such that the bone regions substantially match between the first medical image and the second medical image. For example, in a CT image, since a bone region is extracted with a high brightness as compared to other organ regions, the high-brightness region can be extracted as a bone region by threshold processing. Also, as the method of performing registration to make the bone regions substantially match, a method of performing registration to minimize the distance between surface point groups of bones or a method of generating a distance field image from the outline of a bone region and performing registration between distance field images can be applied.

### (S2050: Associate Plurality of Groups of Bones in First Medical Image with Deformed Image)

In step S2050, the association unit 201 associates the information of a group of bones set in one of the first medical image and the second medical image with the deformed image of the other medical image. The association unit 201 associates the regions of the plurality of groups of bones in one medical image, which are classified based on the identification information of the one medical image, with regions in the other medical image. In this embodiment, processing of associating the information of the plurality of groups of bones in the first medical image with bone regions in the deformed image of the second medical image by the association unit 201 will exemplarily be described. A more detailed method will be described below.

The association unit 201 generates a deformed image by deforming the other medical image using the displacement field generated in the registration, and associates each of regions of the plurality of groups of bones in one medical image with corresponding regions in the deformed image. First, the association unit 201 specifies a region in the deformed image corresponding to each of the regions of the plurality of groups of bones in the first medical image and calculates the difference of image feature information between the regions. More specifically, regions of a plurality of bone groups G1 to G5 in the first medical image shown in Fig. 4 are defined as regions R1_{G1} to R1_{G5}, respectively. The association unit 201 specifies regions R2_{G1} to R2_{G5} in the deformed image corresponding to the regions R1_{G1} to R1_{G5}. The correspondence between the regions R1_{G1} to R1_{G5} and the regions R2_{G1} to R2_{G5} can be specified using the displacement field generated as the result of initial registration executed in step S2040.

Next, the association unit 201 obtains differences S_{G1} to S_{G5} of feature information between regions between the regions R1_{G1} to R1_{G5} and the regions R2_{G1} to R2_{G5}. At this time, as the difference of feature information between the images, for example, Sum of Squared Difference (SSD) used as similarity in registration can be applied. However, the difference of feature information between the regions is not limited to this, and a known method such as Sum of Absolute Difference (SAD) can be used.

Using the difference of feature information, the association unit 201 associates the regions of the plurality of groups of bones in one medical image as bone regions in the deformed image. Sequentially from a group of bones in a region where the difference in feature information between the images is small, the association unit 201 associates the regions of a plurality of groups of bones in one medical image (first medical image) with bone regions in the deformed image. For example, if S_{G1} < S_{G4} < S_{G5} < S_{G2} < S_{G3} holds in ascending order of the difference of feature information, the association unit 201 first associates the bone group G1 in the first medical image with a bone region in the second medical image. Here, the association unit 201 extracts bone regions in the second medical image by, for example, a method described in step S2040 as bone regions BR2. More specifically, in the bone regions BR2, the association unit 201 sets a region that overlaps the region R2_{G1} in the deformed image as the region of the bone group G1. Accordingly, of the bone regions in the deformed image, the region of the group of bones associated with the motion of the trunk is associated.

Next, in the bone regions BR2 except the region R2_{G1} already associated with the region of the group of bones, the association unit 201 sets a region overlapping the region R2_{G4} in the deformed image to the region of the bone group G4. Accordingly, of the bone regions in the deformed image, the region of the group of bones associated with the motion of the right thigh bone is associated. The association unit 201 similarly associates the groups of the bone groups G5, G2, and G3 in ascending order of the difference of feature information between the images, thereby sequentially associating the groups of bones in ascending order of the difference of feature information between the images, that is, reliability of association.

At this time, if the difference of feature information between the first medical image and the deformed image is larger than a predetermined value, not all the bone regions BR2 are associated even if the above-described association processing is performed. For example, for the bone group G2 or the bone group G3, if the posture of the arm is largely different between the images, a position deviation may occur in the initial registration. In this case, concerning a position in the bone region BR2, which is not associated yet, among the regions that are not yet associated with the bone region BR2 in the regions R2_{G1} to R2_{G5}, a group of bones in the region closest from the position of the region can be associated. Thus, the processing of the image processing apparatus 200 is executed.

According to this embodiment, identification of a plurality of bones and classification into groups of bones are performed in only one of the first medical image and the second medical image, the groups of bones are associated with the other image, thereby performing registration for each group of bones.

### <Third Embodiment>

In the first embodiment, a configuration in which a plurality of bones are identified from a first medical image and a second medical image, the bones are classified into a plurality of groups of bones, and registration is performed for each group of bones has been described.

In the third embodiment, a configuration in which registration is performed for each group of bones, and then registration is performed for each bone between a plurality of first bones and a plurality of second bones will be described.

The configuration of an image processing system according to this embodiment is the same as in Fig. 1, and a repetitive description thereof will be omitted. Based on first identification information and second identification information, a registration unit 104 associates bone regions identified as identical bone regions between a plurality of first bones and a plurality of second bones. The registration unit 104 performs rigid body registration between a bone region in a first medical image and a bone region in a second medical image which are associated using a first displacement field D_{G1} and second displacement fields D_{G2} to D_{G5} as initial values (initial displacement fields). The registration unit 104 then performs deformation registration capable of setting the degree of freedom of deformation in a bone region between the bone region in the first medical image and the bone region in the second medical image, which have undergone the rigid body registration.

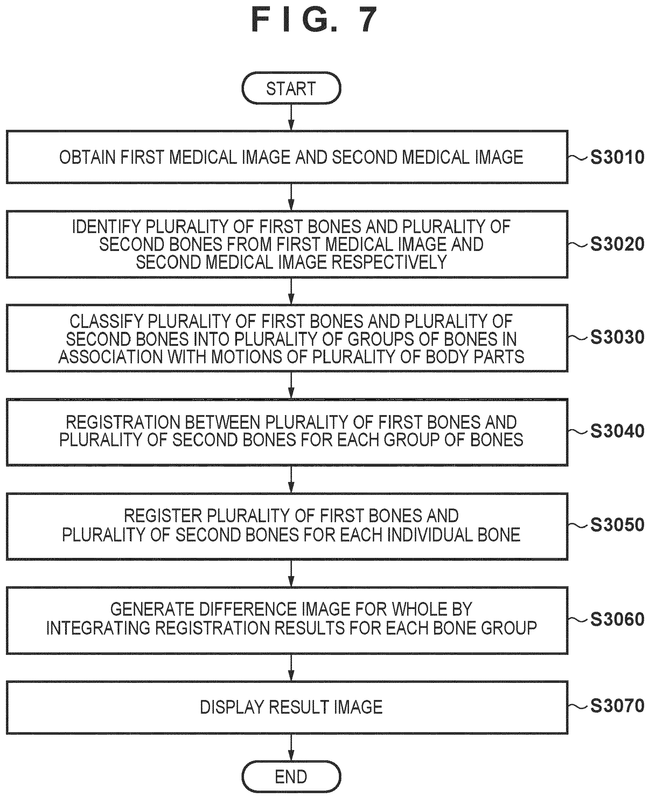

Processing according to this embodiment will be described below with reference to the flowchart of Fig. 7. Note that steps S3010 and S3050 to S3070 in the flowchart showing the overall processing procedure performed by an image processing apparatus 100 are the same as steps S1010 and S 1040 to S 1060 in the first embodiment, and a description thereof will be omitted.

Only differences from the first embodiment will be described below with reference to the flowchart of Fig. 7.

### (S3050: Perform Registration between Plurality of First Bones and Plurality of Second Bones for Each Bone)

In step S3050, using displacement fields (for example, D_{G1} to D_{G5}) between a first medical image and a second medical image, which are generated in step S3040, as initial values (to be referred to as initial displacement fields), the registration unit 104 performs registration for each bone between a plurality of first bones and a plurality of second bones. Then, the registration unit 104 outputs the generated displacement fields (to be referred to as final displacement fields) to an image generation unit 105. Detailed processing will be described below.

First, the registration unit 104 associates bone regions of the same label between the plurality of first bones and the plurality of second bones based on first identification information and second identification information. At this time, as for the unit of individual bones to be associated, the bones are individually identified in a unit that can substantially be regarded as a rigid body. For example, in a case of backbone, each vertebra forming the backbone is identified with a different label. Hence, each bone can substantially be regarded as a rigid body and registered in the following processing.

Second, the registration unit 104 performs rigid body registration using the initial displacement fields between the individual associated bones. For example, the registration unit 104 can perform rigid body registration using the initial displacement fields by the following method. Letting BN1 be a bone in the first medical image and BN2 be a bone in the second medical image, which are the targets of rigid body registration, the registration unit 104 first samples a point group P1 from the region BN1 at a predetermined interval. Next, using an initial displacement field D_{Init} corresponding to a group of bones to which the bone BN1 belongs, the registration unit 104 generates a point group P2 by displacing the point group P1 to a position in the second medical image. Then, using the point group P1 and the point group P2 as corresponding points, the registration unit 104 obtains a rigid body transformation for approximating mutation between the corresponding points by a known rigid body registration method. Thus, rigid body registration can be performed between the first medical image and the second medical image based on the initial displacement field. Similar processing is performed to all associated bones.

Third, the registration unit 104 performs deformation registration between the individual associated bones using the rigid body transformation calculated above as an initial value. Here, the registration unit 104 performs the deformation registration using a known image processing method as the registration method, like step S3040. In step S3040, registration for each group of bones is performed. In this step, however, since the registration target is an individual bone region that can substantially be regarded as a rigid body, a deformation parameter with a smaller degree of freedom of deformation is preferably employed. Deformation registration is performed using the deformation parameter of the smaller degree of freedom of deformation, thereby preventing a bone region from being deformed more than necessary and stabilizing the registration.

For example, in registration using a Free Form Deformation (FFD) model that is a general deformation method, control points in a lattice pattern are arranged in an image, and the positions of the control points are moved, thereby displacing a region existing in the image or a position. If the interval to arrange the control points is large, the degree of freedom of deformation is small. If the interval is small, the degree of freedom of deformation is large. In this embodiment, for example, the registration unit 104 sets a parameter p for controlling the interval to arrange the control points to p times (2 ≤ p) as compared to step S3040, thereby performing deformation registration while making the degree of freedom of deformation small. Note that the setting of the parameter p is an example, and the deformation registration can also be performed while making the degree of freedom of deformation large.

As described above, in this step, the registration unit 104 temporarily performs rigid body registration for individual bones between the images based on the initial displacement field for each group of bones, and then performs deformation registration.

On the other hand, in the registration of step S3050, if the degree of freedom of deformation is set high to appropriately express deformation as a group of bones, a bone region including lesion may forcibly be deformed.

If the registration of this step is performed, the whole bones in the group of bones are appropriately registered, and registration can be performed while maintaining the shape of a bone region including lesion without forcibly deforming it. Thus, the processing of the image processing apparatus 100 is executed.

According to this embodiment, after registration for each group of bones is performed, registration is performed for individual bones between the plurality of first bones and the plurality of second bones, thereby more correctly registering the bones between the images.

### <Fourth Embodiment>

In the first embodiment, a configuration in which a plurality of bones are identified from a first medical image and a second medical image, the bones are classified into a plurality of groups of bones, and registration is performed for each group of bones has been described.

In the fourth embodiment, a configuration in which it is determined whether there is a high possibility that groups of bones of limbs are normally classified, groups of bones of limbs for which it is determined that these may not be normally classified are reclassified into groups of the bones of the trunk, and after that, registration is performed will be described.

In this embodiment, determining whether there is a high possibility that groups of bones of limbs are normally classified is performed based on determining whether a predetermined transplant such as an artificial bone exists in or near a group of bones of limbs or whether there exists a bone recognition region in which the bone extraction reliability does not satisfy a predetermined threshold. The bone recognition region in which the bone extraction reliability does not satisfy a predetermined threshold will sometimes be referred to as a low-reliability bone region or a bone extraction error region.

For a group of bones of limbs that are not normally classified, the bones of the trunk and the bones of the limbs may not normally be classified, and registration performance may lower. According to this embodiment, for the group of bones of the limbs determined not to be normally classified at high possibility, registration is performed without separating these from the bones of the trunk, thereby suppressing lowering of registration accuracy caused by separation of the bones of the limbs.

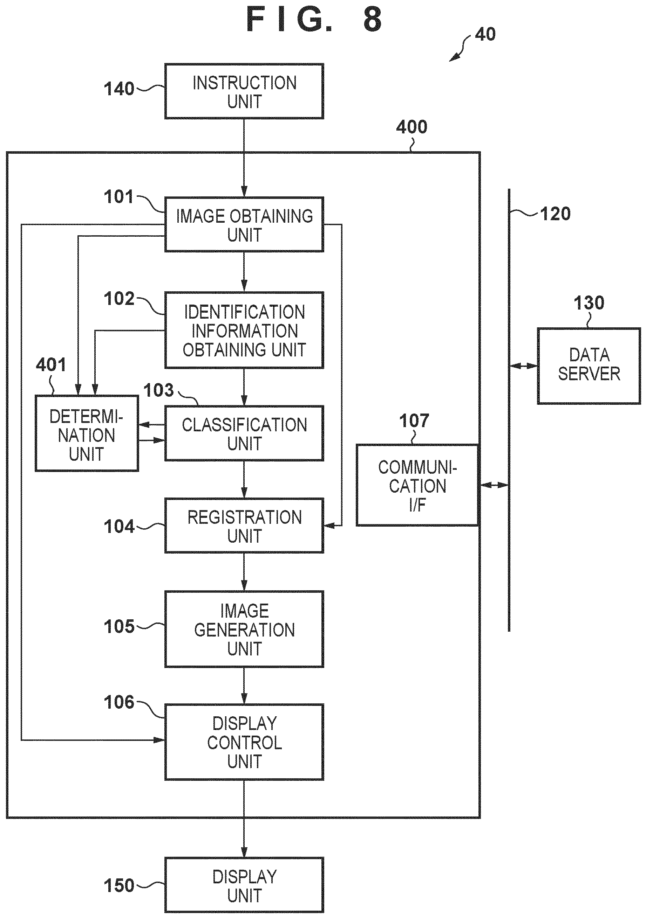

Fig. 8 is a view showing the configuration of an image processing system 40 according to the fourth embodiment. An image processing apparatus 400 according to the fourth embodiment includes a determination unit 401 as a constituent element. The configuration other than the determination unit 401 and a classification unit 103 is the same as the configuration of the image processing apparatus 100 described with reference to Fig. 1, and a description thereof will be omitted.

The determination unit 401 obtains determination information that determines whether there is a high possibility that the groups of bones of the limbs classified by the classification unit 103 are normally classified. The classification unit 103 reclassifies the groups of bones of the limbs to the group of bones of the trunk based on the determination information obtained by the determination unit 401.

The determination unit 401 determined whether a second bone group classified in at least one of a first medical image and a second medical image includes at least one of a transplant and a bone recognition region in which the bone extraction reliability does not satisfy a predetermined threshold. If the result of determination of the determination unit 401 indicates that at least one of a transplant and a bone recognition region in which the bone extraction reliability does not satisfy a predetermined threshold is included, the classification unit 103 reclassifies bones belonging to the second bone group to a first bone group.

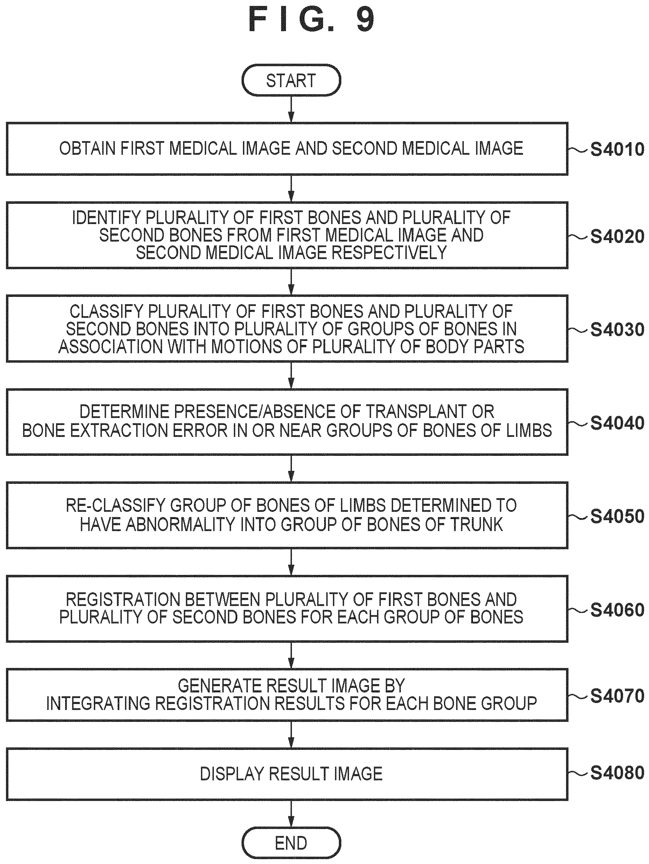

Processing according to this embodiment will be described below with reference to the flowchart of Fig. 9. Note that steps S4070 and S4080 in the flowchart showing the overall processing procedure performed by the image processing apparatus 400 are the same as steps S1050 and S1060 in the first embodiment, and a description thereof will be omitted.

Only differences from the first embodiment will be described below with reference to the flowchart of Fig. 9.

### (S4010: Obtain First Medical Image and Second Medical Image)

In step S4010, an image obtaining unit 101 obtains a first medical image and a second medical image, as in the first embodiment. The image obtaining unit 101 outputs the first medical image and the second medical image to an identification information obtaining unit 102, the determination unit 401, a registration unit 104, and a display control unit 106.

### (S4020: Identify Plurality of First and Second Bones from Each of Two Images)

In step S4020, the identification information obtaining unit 102 obtains at least one of first identification information and second identification information, as in the first embodiment. The identification information obtaining unit 102 then outputs the at least one of the first identification information and the second identification information to the classification unit 103 and the determination unit 401.

### (S4030: Classify Plurality of First Bones and Plurality of Second Bones into Plurality of Groups of Bones)

In step S4030, the classification unit 103 classifies a plurality of first bones and a plurality of second bones into a plurality of groups of bones, as in the first embodiment. The classification unit 103 outputs the information of the plurality of classified groups of bones to the determination unit 401 and the registration unit 104.

### (S4040: Determine Whether There Is High Possibility That Groups of Bones of Limbs Are Normally Classified)

In step S4040, the determination unit 401 obtains first determination information that determines, based on the first medical image and the information of the plurality of groups of bones, whether there is a high possibility that a second bone group is normally classified in the first medical image. The determination unit 401 also obtains second determination information that determines, based on the second medical image and the information of the plurality of groups of bones, whether there is a high possibility that the second bone group is normally classified in the second medical image. The determination unit 401 then outputs the obtained first determination information and the second determination information to the classification unit 103.

In this embodiment, the first determination information and the second determination information are information representing whether there is a high possibility that the information of the groups of bones of limbs includes an abnormality. If a transplant such as an artificial bone exists in the body of a subject, there is a possibility that identification information obtained in step S4020 is not normally obtained (a label different from an original bone is identified) because of degradation of image quality around the transplant due to the influence of metal artifact or the like. In this embodiment, the determination unit 401 determines the presence/absence of a predetermined transplant in the groups of bones of limbs, if a transplant exists, judges that there is a high possibility that the information of the groups of bones of limbs includes an abnormality, and obtains determination information representing the presence of an abnormality.

An example of a detailed processing method will be described below. The determination unit 401 determines whether a transplant is included in each of groups G2 to G5 of bones that move in association with the motions of limbs in the first medical image, and obtains first determination information.

More specifically, if pixels having pixel values not less than a predetermined threshold exist in a predetermined volume or more in a bone region identified as one of the groups G2 to G5 of bones of limbs in the first medical image or in a region around that, the determination unit 401 determines that a transplant exists in the group. If a transplant exists, the accuracy of identification information may lower. For this reason, the determination is preferably done using not only the pixels in the first medical image identified as the groups G2 to G5 of bones of limbs but also pixel values in the periphery.

For example, if a right thigh bone BL13 is classified into the bone group G4, the presence/absence of a transplant in the bone group G4 may be determined using pixels in a rectangular region formed by overlap of the bounding box of the right thigh bone BL13 and the bounding box of an ilium BL11 adjacent to that. Note that the determination of the presence/absence of a transplant can thus be performed only for a target region near the boundary portion between the group G2 (or G3, G4, or G5) of bones of limbs a first bone group G1. Alternatively, the determination may be performed using, as the determination target region, an entire region including each of the groups G2 to G5 of bones of limbs. Here, the predetermined threshold may be, for example, a CT value (for example, 6000H.U.) corresponding to a metal artificial bone or may be a CT value corresponding to another transplant such as a pacemaker existing near a limb bone. Also, the predetermined volume may be a value corresponding to the volume of a predetermined general transplant (for example, an artificial thigh bone), or may be a predetermined constant (for example, 1 pixel).

For example, if it is determined that the bone groups G2 and G3 in the first medical image do not include any transplant, the determination unit 401 obtains, as the first determination information, determination information representing that there is no abnormality concerning the bone groups G2 and G3. If it is determined that the bone groups G4 and G5 in the first medical image include transplants, the determination unit 401 obtains determination information representing that there is an abnormality concerning the bone groups G4 and G5, and obtains the determination information in each group of bones as the first determination information.

In this embodiment, the processing of determining the presence/absence of a transplant is not limited to processing using the threshold of pixel values. The presence/absence of a transplant may be determined using a known method. For example, a transplant may be identified by a region extraction method using known machine learning. In this case, a learned inference model that has learned the label regions of transplants of many cases in advance may be constructed, and the label region of a transplant may be extracted by inputting a target medical image to the inference model. As the region extraction method using known machine learning, for example, a method such as a Convolutional Neural Network (CNN) can be used. Note that transplant region extraction need not always be performed, and an inference model that infers the presence/absence of a transplant in a predetermined range in an image may be used. Also, the presence/absence of a transplant need not always be determined by analyzing the first medical image and the second medical image, and information (for example, information of a surgery history concerning artificial joints included in medical chart information or the like) about the presence/absence of a transplant in the first medical image and the second medical image may be obtained from a data server 130.

Note that the determination unit 401 may obtain, as the first determination information, information different from the above-described information about the presence/absence of a transplant. For example, the determination unit 401 may determine the presence/absence or degree of an identification error in the first identification information obtained by the identification information obtaining unit 102 and obtain this as the first determination information.

More specifically, for example, the degree of divergence between the label region of a bone of limbs obtained as the first identification information and the statistical shape model of the bone is measured, and if the degree of divergence is larger than a predetermined threshold, it may be determined that the first identification information includes an abnormality. As for the degree of divergence, the shape of the label region of the bone of limbs obtained as the first identification information and the statistical shape model of the bone registered to the shape are each obtained as a binary image, and a known index such as a DICE coefficient representing the degree of matching between the two regions can be used. That is, if the calculated degree of matching is equal to or less than a threshold, it can be determined that the degree of divergence is large, and an abnormality (identification error) exists.

The above-described degree of divergence can be discussed by replacing it with an extraction reliability obtained by normalizing the degree of divergence and calculating the difference from 1. Based on a determination result representing that the extraction reliability does not satisfy a predetermined threshold in the extraction reliability (the extraction reliability is less than the threshold), the determination unit 401 may determine that the first identification information includes an abnormality.

The determination unit 401 may determine that the first identification information includes an abnormality by determining whether at least one of a first determination result representing that a transplant is included in the second bone group in at least one of a first image and a second image and a second determination result representing that a bone recognition region that does not have a predetermined extraction reliability is included in the second bone group is satisfied.

Alternatively, information representing whether an anatomically abnormal shape occurs, for example, whether a cavity exists in the label region of a bone of limbs or whether one bone is separated to two label regions may be used as the determination information. Whether an abnormality exists may be determined using the positional relationship between the label regions of bones. For example, if the position of the center of gravity of the label region of the right thigh bone BL13 is located on the head side with respect to the position of the center of gravity of the label region of a backbone BL10, it can be determined that the information of the group of bones to which the right thigh bone belongs includes an abnormality.

Note that the second determination information that is the determination information about the second bone group in the second medical image can also be obtained by the same method as the first determination information.

### (S4050: Reclassify Groups of Bones of Limbs Determined to Include Abnormality to Group of Bones of Trunk)

In step S4050, the classification unit 103 reclassifies the plurality of first bones and the plurality of second bones into a plurality of groups of bones based on at least one of the first determination information and the second determination information obtained in step S4040. The classification unit 103 then outputs the information of the plurality of reclassified groups of bones to the registration unit 104.

Using the identification information obtained by the identification information obtaining unit 102 and information about the result of determination of the determination unit 401, the classification unit 103 reclassifies a bone belonging to the second bone group and determined to include an abnormality into the first bone group. If it is determined that one of the groups G2 to G5 of bones of limbs includes an abnormality, the classification unit 103 reclassifies the group to which the limb determined to be abnormal belongs such that it is included in the bone group G1 of the trunk. At this time, the group of the limb with the abnormality may be obtained based on one of the first determination information and the second determination information or may be obtained based on both. If the group is obtained based on both pieces of information, for example, the classification unit 103 reclassifies the group of bones of limbs determined to include a transplant or an abnormality based on at least one of the first determination information and the second determination information such that it is included in the bone group G1 of the trunk.

For example, if images obtained by capturing images of the same subject at different times are processed as the first medical image and the second medical image, there is rarely a situation in which an artificial bone exists in the medical image on the past side in the elapse of time, but the artificial bone does not exist in the medical image on the future side in the elapse of time. Thus, as a form considering a use case concerning the history of introduction of a transplant including an artificial bone to the subject of interest, a form in which the classification unit 103 determines an abnormality in the groups of limbs using only the determination information of the medical image on the past side with an older image capturing time is a modification of this embodiment. Note that if an abnormality is determined based on only one piece of information (for example, the first determination information), in the processing of the determination unit 401 in step S4040, only the determination information (for example, the first determination information) to be used is obtained, and the determination information (for example, the second determination information) that is not used need not be obtained. The determination unit 401 may employ an adaptive implementation form in which the above-described determination operation of abnormality determination is performed based on whether a transplant is included in the second bone groups of the first image and the second image.

Note that if a plurality of groups of bones of limbs exist, not a configuration in which only the group of bones of limbs determined to include an abnormality is reclassified and included in the group G1 but a configuration in which other groups are also reclassified and included in the group G1 may be employed. As an example of the latter, for example, artifacts caused by a transplant may occur in a wide range. Hence, if an abnormality exists in the group to which the right thigh bone BL13 is classified, not only the group but also a group to which a left thigh bone BL14 is classified may be reclassified and included in the group G1 of bones of the trunk.

### (S4060: Perform Registration between Plurality of First Bones and Plurality of Second Bones for Each Group of Bones)

In step S4060, the registration unit 104 performs registration between the plurality of first bones and the plurality of second bones for each group of bones, and outputs a generated displacement field to an image generation unit 105.

In this embodiment, the image generation unit 105 obtains, as an image I1_{G1}, an image obtained by replacing pixel values in the bone regions of the groups of bones of limbs determined not to include an abnormality with a predetermined pixel value (for example, a pixel value close to a peripheral tissue outside the bones) from the first medical image. Since this makes it possible to generate the image of each group without cutting out the group of bones of limbs determined to include an abnormality, cutout processing can be prevented from failing due to an abnormality in the information of the group of bones. It is possible to suppress lowering of registration accuracy as compared to a case where registration is performed using an image obtained by failed cutout processing.

According to this embodiment, if an abnormality exists in the identification information of a body part that makes a different motion, registration is performed without separating the part with the abnormality from the trunk portion. It is therefore possible to suppress lowering of registration accuracy of the part with the abnormality as compared to the first embodiment. Concerning a part without any abnormality, in a case where the motions of a plurality of bones belonging to different body parts are largely different between a plurality of medical images, or discontinuous deformation occurs in the boundary portion, registration accuracy can be prevented from lowering, as in the first embodiment. That is, according to this embodiment, it is possible to correctly and stably perform registration between images.

### (Modification 1)

In the fourth embodiment, in step S4040, the determination processing is performed for the groups of bones of limbs. However, the determination processing may be performed for the group of bones of the trunk. For example, in step S4030, if individual bones belonging to the trunk are classified into different groups, the determination unit 401 may perform, for each group of bones, the determination of a transplant and a bone recognition region in which bone extraction reliability does not satisfy a predetermined threshold. For example, if one or a plurality of vertebrae of the backbone are classified into different groups, in step S4040, the determination unit 401 performs the determination processing for each vertebra group. Then, in step S4050, the classification unit 103 reclassifies a group of vertebrae determined to include an abnormality into the group G1 of bones of the trunk for which cutout processing is to performed. Thus, it is possible to suppress lowering of registration accuracy due to an abnormality in the information of the groups of bones of the trunk, like the bones of limbs.

### (Modification 2)

In the fourth embodiment, in step S4040, the determination unit 401 obtains determination information as information about the result of determination for the groups of bones of limbs. However, the target to obtain determination information is not necessarily the groups of bones. For example, the determination unit 401 may obtain the first determination information and the second determination information using the first identification information and the second identification information obtained in step S4020. The determination unit 401 may be configured to determine, for the label regions of the bones of limbs in the first identification information and the second identification information, whether a transplant exists, or whether a bone recognition region in which bone extraction reliability does not satisfy a predetermined threshold exists, as in step S4040, and obtain the first determination information and the second determination information.

In this case, the process of step S4030 may be skipped, and in step S4050, the classification unit 103 may classify the plurality of groups of bones based on the first identification information, the second identification information, the first determination information, and the second determination information. Using the identification information (the first identification information and the second identification information) obtained by the identification information obtaining unit 102 and the information about the result of determination (the first determination information and the second determination information), the classification unit 103 reclassifies a bone belonging to the second bone group determined to include an abnormality into the first bone group. That is, the classification unit 103 classifies a bone of limbs with an abnormality such that it is included in the group of bones of the trunk, and classifies the remaining bones of limbs into the second bone group, as in the first embodiment. According to this, since the process of step S4030 can be omitted, the processing can efficiently be executed.

### (Modification 3)

In the fourth embodiment, in step S4020, the identification information obtaining unit 102 may obtain the label region of a transplant as the first identification information and the second identification information. For example, in addition to identification of bones, identification of a transplant may be performed by a region extraction method using known machine learning. In step S4040, the determination unit 401 may determine, based on the first identification information and the second identification information including the label region of the transplant obtained in step S4020, whether the groups of bones of limbs include a transplant.

### <Fifth Embodiment>

In the first embodiment, a configuration in which a plurality of bones are identified from a first medical image and a second medical image, the bones are classified into a plurality of groups of bones, and registration is performed for each group of bones has been described.

In this embodiment, a configuration in which before registration is performed for each group of bones, for the groups of bones of limbs, initial registration is performed for each bone (to be referred to as a partial bone) in the groups of bones, and initial registration results are integrated will be described.

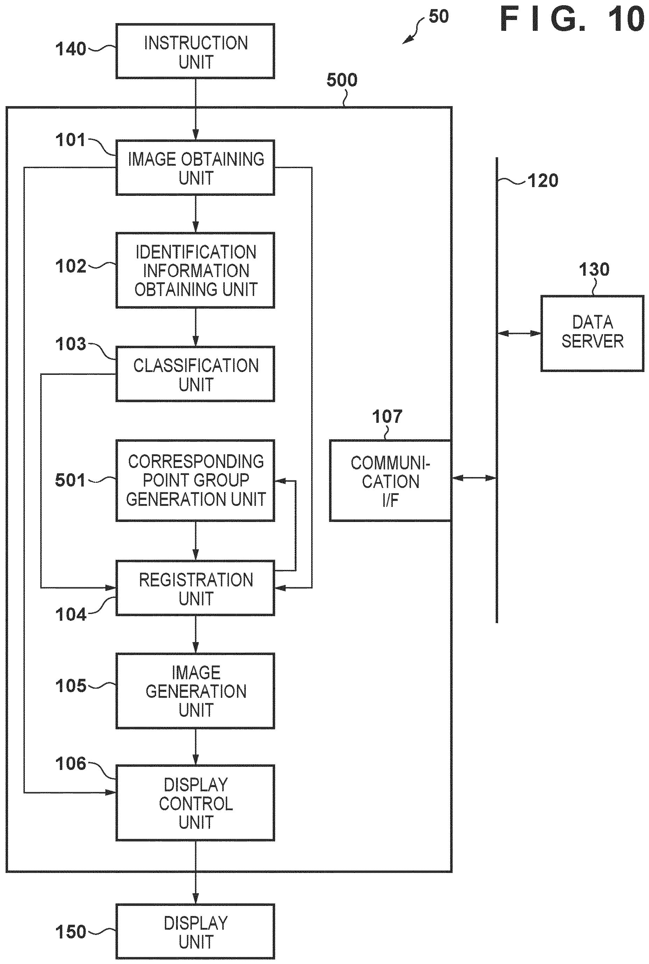

Fig. 10 is a view showing the configuration of an image processing system 50 according to the fifth embodiment. An image processing apparatus 500 according to the fifth embodiment includes a corresponding point group generation unit 501 as a constituent element.

The configuration other than the corresponding point group generation unit 501 is the same as in Fig. 1, and a description thereof will be omitted. The corresponding point group generation unit 501 performs processing of generating a corresponding point group based on the initial registration result for a plurality of input partial bones.

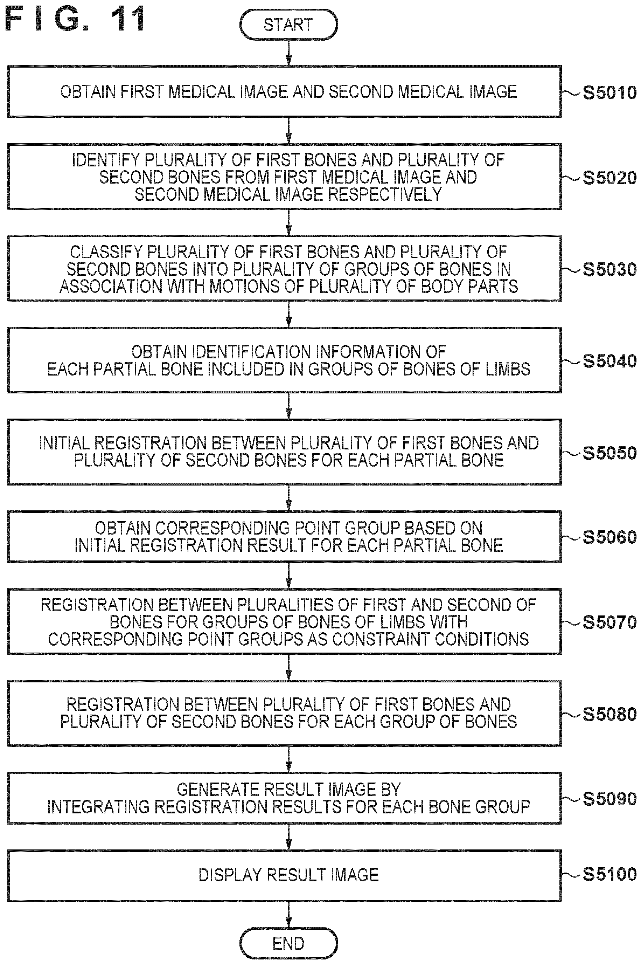

Processing according to this embodiment will be described below with reference to the flowchart of Fig. 11. Note that steps S5010 to S5030 and S5090 to S5100 in the flowchart showing the overall processing procedure performed by the image processing apparatus 500 are the same as steps S1010 to S1030 and S1050 to S1060 in the first embodiment, and a description thereof will be omitted.

Only differences from the first embodiment will be described below with reference to the flowchart of Fig. 11.

### (S5040: Obtain Identification Information of Partial Bones Included in Groups of Bones of Limbs)

In step S5040, based on the information of the groups of bones obtained from a classification unit 103, a registration unit 104 obtains identification information of a partial bone included in a second bone group (to be also referred to as the groups of bones of limbs hereinafter) that moves in association with the motions of the limbs. The registration unit 104 obtains identification information of a partial bone belonging to a plurality of bones classified into the groups of bones of limbs in a first medical image and identification information of a partial bone belonging to a plurality of bones classified into the groups of bones of limbs in a second medical image. That is, based on the information obtained from the classification unit 103, the registration unit 104 obtains first identification information of a first partial bone for identifying the first partial bone in a plurality of first bones, which is included in the plurality of first bones depicted in the first medical image and classified into the second bone group, and first identification information of a second partial bone for identifying the second partial bone different from the first partial bone. Also, the registration unit 104 obtains second identification information of the first partial bone for identifying the first partial bone which is included in a plurality of second bones depicted in the second medical image and classified into the second bone group, and second identification information of the second partial bone for identifying the second partial bone. Detailed processing will be described below.

In this step, the registration unit 104 obtains the identification information of partial bones belonging to groups G2, G3, G4, and G5 of bones of limbs. A case where a plurality of partial bones exist in a group will be described with reference to Fig. 4.

In Fig. 4, a right upper arm bone BL2 and a right scapula bone BL6, which move in association with the motion of the right arm, exist in the group G2 of bones of a right arm. At this time, the registration unit 104 obtains identification information of the right upper arm bone BL2 that is a first partial bone, and identification information of the right scapula bone BL6 that is a second partial bone. For the group G3 of bones of a left arm as well, the registration unit 104 obtains identification information of a left upper arm bone BL3 that is a first partial bone, and identification information of a left scapula bone BL7 that is a second partial bone.

Note that as described in the first embodiment, if the classification unit 103 classifies a right collar bone BL4 into the group G2, the registration unit 104 further obtains identification information of the right collar bone BL4 that is a third partial bone in the group G2. Similarly, if a left collar bone BL5 is classified into the group G3, the registration unit 104 further obtains identification information of the left collar bone BL5 that is a third partial bone in the group G5.

If only one partial bone exists in a group, the registration unit 104 obtains the partial bone as a first partial bone. For example, in the group G4 of bones of a right leg in Fig. 4, the registration unit 104 obtains identification information of a right thigh bone BL13 that is a first partial bone, and in the group G5 of bones of a left leg, the registration unit 104 obtains identification information of a left thigh bone BL14 that is a first partial bone.

### (S5050: Perform Initial Registration between Plurality of First Bones and Plurality of Second Bones)

In step S5050, the registration unit 104 performs initial registration between the plurality of first bones and the plurality of second bones for each partial bone based on the identification information of the partial bones included in the plurality of first bones in the first medical image obtained in step S5040 and the identification information of the partial bones included in the plurality of second bones in the second medical image. The registration unit 104 performs initial registration of the first partial bone based on the first identification information of the first partial bone and the second identification information of the first partial bone between the first medical image and the second medical image. In addition, the registration unit 104 performs initial registration of the second partial bone based on the first identification information of the second partial bone and the second identification information of the second partial bone. The registration unit 104 then outputs initial transformation information generated by performing initial registration to the corresponding point group generation unit 501. Detailed processing will be described below.

In this step, the registration unit 104 extracts the surface point group of each partial bone based on the first identification information representing the partial bones belonging to the plurality of first bones. More specifically, the registration unit 104 extracts the surface point group of each partial bone from a label region on a label image that is the first identification information using a known point group sampling method. The extracted surface point group will be referred to as a first surface point group SP1. The registration unit 104 performs the processing of extracting a surface point group for each partial bone in the groups of bones of limbs. Thus, for example, concerning the group G2 of the bones of the right arm, a first surface point group SP1_{BL2} of the first partial bone (right upper arm bone BL2) and a first surface point group SP1_{BL6} of the second partial bone (right scapula bone BL6) can be obtained.

Similarly, the registration unit 104 samples a surface point group from a label region representing partial bones belonging to the plurality of second bones, thereby obtaining a second surface point group SP2 concerning each partial bone. Thus, for example, concerning the group G2 of the bones of the right arm, a second surface point group SP2_{BL2} of the first partial bone (right upper arm bone BL2) and a second surface point group SP2_{BL6} of the second partial bone (right scapula bone BL6) can be obtained.

Next, the registration unit 104 performs initial registration between corresponding partial bones using the first surface point group SP1 and the second surface point group SP2. As the method of initial registration using the surface point groups, for example, an ICP algorithm that performs rigid body registration for minimizing the distance between surface point groups can be used. The registration unit 104 performs rigid body registration for each partial bone in each group of bones of limbs, thereby obtaining a rigid body transformation matrix as initial transformation information for each partial bone. Thus, for example, concerning the group G2 of the bones of the right arm, the registration unit 104 obtains, as the initial transformation information, a rigid body transformation matrix T_{BL2} that makes the first partial bone (right upper arm bone BL2) in the first image substantially match the partial bone (right upper arm bone BL2) in the second image. Similarly, the registration unit 104 obtains, as the initial transformation information, a rigid body transformation matrix T_{BL6} that makes the second partial bone (right scapula bone BL6) in the first image substantially match the partial bone (right scapula bone BL6) in the second image.

As another method of performing initial registration, a known method of performing rigid body registration between a label image that is the first identification information representing the partial bones in the first image and a label image that is the second identification information representing the partial bones in the second image using an image similarity or the degree of matching between regions may be used.

The initial transformation information obtained by initial registration is not necessarily a rigid body transformation matrix. For example, an affine transformation matrix with a higher degree of freedom of deformation may be used. This can be implemented by changing transformation information to be sequentially updated by the ICP algorithm from a rigid body transformation matrix to an affine transformation matrix in the above-described example.

### (S5060: Obtain Corresponding Point Group Based on Initial Registration Result for Each Partial Bone)

In step S5060, the corresponding point group generation unit 501 obtains a corresponding point group concerning the second bone groups (groups of bones of limbs) between the first medical image and the second medical image based on initial transformation information obtained by performing initial registration. The corresponding point group generation unit 501 then outputs information of the obtained corresponding point group to the registration unit 104. Detailed processing will be described below.

In this step, first, the corresponding point group generation unit 501 performs sampling in the region of a partial bone in one of the first medical image and the second medical image, thereby obtaining pieces of discrete position information in the region as a point group. In this embodiment, the thus obtained point group will be referred to as a sampling point group. When performing sampling in the region of a partial bone on the first medical image, for example, sampling is performed at an equal interval (for example, 3 mm interval) in the label region of a label image that is the identification information of the partial bone on the first medical image. Note that the interval of sampling is not limited to the equal interval and may arbitrarily be set.

The corresponding point group generation unit 501 performs sampling for each partial bone in the groups of bones of limbs and obtains a sampling point group P1 of each partial bone. For example, the corresponding point group generation unit 501 obtains a sampling point group P1_{BL2} sampled on the first partial bone (right upper arm bone BL2) in the first image, and a sampling point group P1_{BL6} sampled on the second partial bone (right scapula bone BL6) in the first image. Note that the target to obtain the sampling point group may be a partial bone in the second image.

Next, based on the initial transformation information obtained in step S5050, the corresponding point group generation unit 501 obtains a sampling point group in which the coordinates of the sampling point group obtained for a partial bone in one of the first medical image and the second medical image are transformed into the position of the partial bone in the other medical image. The corresponding point group generation unit 501 obtains a corresponding point group concerning the first partial bone and a corresponding point group concerning the second partial bone between the first medical image and the second medical image.

Here, the corresponding point group generation unit 501 obtains a sampling point group P2 in which the coordinates of the sampling point group P1 of a partial bone in the first image are transformed into the position of the partial bone in the second image. Then, the corresponding point group generation unit 501 generates a corresponding point group CP that associates the sampling point group P1 before transformation with the sampling point group P2 after transformation. The corresponding point group generation unit 501 performs the same processing for each partial bone in the groups of bones of limbs, thereby obtaining corresponding point group of each partial bone. For example, the corresponding point group generation unit 501 generates a sampling point group P2_{BL2} after transformation in which the sampling point group P1_{BL2} of the first partial bone (right upper arm bone BL2) in the first image is transformed into the coordinates of the partial bone (right upper arm bone BL2) in the second image using the rigid body transformation matrix T_{BL2} that is the initial transformation information. Also, the corresponding point group generation unit 501 generates a sampling point group P2_{BL6} after transformation in which the sampling point group P1_{BL6} of the second partial bone (right scapula bone BL6) on the first image is transformed into the coordinates of the partial bone (right scapula bone BL6) in the second image using the rigid body transformation matrix T_{BL6} that is the initial transformation information.

The corresponding point group generation unit 501 generates a corresponding point group CP_{BL2} that associates the points of the sampling point group P1_{BL2} with those of the sampling point group P2_{BL2}. In addition, the corresponding point group generation unit 501 generates a corresponding point group CP_{BL6} that associates the points of the sampling point group P1_{BL6} with those of the sampling point group P2_{BL6}. Note that the direction to transform the sampling point group may be the direction from the second medical image to the first medical image. In this case, the coordinates of the sampling point group obtained from a partial bone in the second medical image are transformed using reverse transformation of initial transformation information obtained in step S5050 (in a case of a rigid body transformation matrix, an inverse matrix), thereby obtaining a sampling point group transformed to the coordinates of the partial bone in the first medical image.

Next, the corresponding point group generation unit 501 generates an integrated corresponding point group CP_{I} in which the corresponding point groups of each partial bone belonging to a group of bones are integrated for each of the groups of bones of limbs. The corresponding point group generation unit 501 can implement integration of corresponding point groups by putting the corresponding point groups of each partial bone together. The corresponding point group generation unit 501 obtains an integrated corresponding point group in which the corresponding point group concerning the first partial bone and the corresponding point group concerning the second partial bone are put together. For example, in the group G2 of bones of the right arm, the corresponding point group generation unit 501 puts the corresponding point groups CP_{BL2} (N_{BL2} pairs) of the first partial bone (right upper arm bone BL2) belonging to the group G2 and the corresponding point groups CP_{BL6} (N_{BL6} pairs) of the second partial bone (right scapula bone BL6) together, thereby generating integrated corresponding point groups CP_{I_G2} ((N_{BL2} + N_{BL6}) pairs). The corresponding point group generation unit 501 performs the same processing for the remaining groups G3 to G5 of limbs, thereby obtaining integrated corresponding point groups CP_{I_G3} to CP_{I_G5}.

Note that the obtaining of the corresponding point group in this step need not always be executed for a group of bones of limbs which includes only one partial bone (for example, G4 (right thigh bone) or G5 (left thigh bone)).

### (S5070: Perform Registration between Plurality of First Bones and Plurality of Second Bones of Groups of Bones of Limbs Using Corresponding Point Group as Constraint Condition)

In step S5070, the registration unit 104 performs registration between the plurality of first bones and the plurality of second bones of the groups of bones of limbs using the integrated corresponding point group obtained in step S5060 as a constraint condition, and obtains a displacement field. More specifically, under a constraint condition that the corresponding point groups that form the integrated corresponding point group are made to substantially match, the registration unit 104 obtains deformation for smoothly transforming positions other than those. The registration unit 104 performs deformation processing using the corresponding point group concerning the first partial bone and the corresponding point group concerning the second partial bone as constraint conditions, thereby obtaining integrated transformation information (to be referred to as integrated initial transformation information hereinafter) in which initial transformation information generated by initial registration of the first partial bone and initial transformation information generated by initial registration of the second partial bone are put together. The registration unit 104 then performs second registration between the plurality of first bones and the plurality of second bones based on the integrated initial transformation information. That is, the registration unit 104 integrates the initial transformation information of partial bones in the groups of bones of limbs and obtains integrated initial transformation information that re-expresses one displacement field for each of the groups of bones of limbs. Detailed processing will be described below.

In this step, the registration unit 104 performs registration for calculating the correspondence relationship of continuous spaces using the correspondence relationship between discrete positions defined as the integrated corresponding point groups CP_{I_G2} to CP_{I_G5} for the groups G2 to G5 of bones of limbs as the constraint condition. At this time, as the registration method, a known method using corresponding point groups (landmarks) can be used. For example, as an image deformation model, a known deformation model such as Thin Plate Spline (TPS) can be used, in addition to the Free Form Deformation (FFD) model and the Demons algorithm described concerning step S1040. The registration unit 104 thus obtains pieces of integrated initial transformation information ID_{G2} to ID_{G5}.

Note that the registration in this step need not always be executed for a group of bones of limbs which includes only one partial bone (for example, G4 (right thigh bone) or G5 (left thigh bone)). That is, without executing registration in this step, the initial transformation information obtained in step S5050 may directly be applied as integrated initial transformation information. In this case, using a rigid body transformation matrix T_{BL13} and a rigid body transformation matrix T_{BL14} obtained as initial transformation information, the registration unit 104 obtains integrated initial transformation information ID_{G4} = rigid body transformation matrix T_{BL13}, and integrated initial transformation information ID_{G5} = rigid body transformation matrix T_{BL14}.

### (S5080: Perform Registration between Plurality of First Bones and Plurality of Second Bones for Each Group of Bones)

In step S5080, the registration unit 104 performs registration between the plurality of first bones and the plurality of second bones for each group of bones. At this time, concerning the group G1 of bones of the trunk, the same registration as in step S 1040 is performed, and a description thereof will be omitted. On the other hand, concerning the groups G2 to G5 of bones of limbs, the registration unit 104 performs registration between the plurality of first bones and the plurality of second bones using the integrated initial transformation information obtained by the process of step S5070 as an initial value. Detailed processing will be described below.

The registration unit 104 performs registration for each corresponding group between images I1_{G2} to I1_{G5} cut out from a first medical image I1 and images I2_{G2} to I2_{G5} cut out from a second medical image 12. At this time, first, registration unit 104 generates images (initial deformed images) I2_{ID_G2} to I2_{ID_G5} by performing initial deformation of the images I2_{G2} to I2_{G5} that are floating images in this embodiment using the pieces of integrated initial transformation information ID_{G2} to ID_{G5}.

Next, the registration unit 104 generates displacement fields D_{G2} to D_{G5} for further deforming the initial deformed images I2_{ID_G2} to I2_{ID_G5} and making these match the images I1_{G2} to I1_{G5} that are reference images. This is the same as the process of step S 1040 except that the floating images I2_{G2} to I2_{G5} in step S1040 are replaced with the initial deformed images I2_{ID_G2} to I2_{ID_G5}, and a description thereof will be omitted.

According to this embodiment, it is possible to obtain a transformation result in which partial bones in the same group match between images. Particularly, in a case where the posture between the images changes on a partial bone basis, more accurate initial transformation information can be obtained as compared to a case where initial transformation of the group is expressed by single rigid body transformation. When processing from step S5080 is executed using the thus obtained initial transformation information as an initial value, registration between images can be executed more stably at higher accuracy.

### (Modification 1)

A case where processing from step S5050 to step S5070 in the fifth embodiment is executed as registration processing in the groups of bones classified in step S5030 has been described as an example. However, the technique of the disclosure is not limited to this. For example, regardless of grouping of bones, registration concerning a plurality of partial bones that are adjacent to each other may be executed. For example, as shown in Fig. 4, registration processing may be executed for the right upper arm bone BL2 and the right scapula bone BL6, which are adjacent to each other, or registration processing may be executed for a backbone BL10 and a rib BL8.

As detailed processing, the processes of steps S5030 and S5040 are omitted, and a plurality of arbitrary adjacent bones identified in step S5020 are defined as partial bones of the processing target. The registration unit 104 executes the processes of steps S5060 and S5070 for the partial bones of the processing target, thereby obtaining a registration result (one displacement field) concerning the plurality of partial bones of the target.

The registration unit 104 then omits the process of step S5080, and performs, as the process of step S5090, difference processing between the images based on the registration result (displacement field) obtained in step S5070, thereby obtaining the difference image between the plurality of bones of the target. The above-described processing target may be an anatomical structure other than bones. For example, in place of the plurality of partial bones, the right atrium and the left atrium, or the right ventricle and the left ventricle inside the heart, which are considered to be adjacent to each other and have no continuous deformation in the boundary therebetween can be applied to this processing.

### (Modification 2)

In the fifth embodiment, processing of, in step S5070, expressing initial registration of partial bones belonging to the groups of bones of limbs by one displacement field and then, in step S5080, performing registration of the groups of bones of limbs using the displacement field obtained in step S5070 as an initial value has been described.

However, the processes of steps may be executed simultaneously as one process. More specifically, the registration unit 104 may perform registration using matching of the integrated corresponding point groups in step S5070 and the image similarity in step S5080 as constraint conditions.

This registration can be executed by a known method (the Free Form Deformation (FFD) model or the Demons algorithm) described concerning step S5070 or S5080. This makes it possible to execute registration simultaneously considering both the constraint condition of discrete positions by the integrated corresponding point groups and the image similarity. Accordingly, in the processes of steps S5070 and S5080, registration can be performed by making the integrated corresponding point groups match better, as compared to a case where registration is performed in two steps.

Also, in the process of step S5080, the registration unit 104 may perform registration using both matching of the integrated corresponding point groups and the image similarity as constraint conditions, as described above, using the displacement field obtained in step S5070 as an initial value. According to this processing, after registration is roughly performed, detailed registration is executed based on the corresponding point groups and the image similarity. For this reason, fast and accurate registration can be performed.

According to the technique of the disclosure, it is possible to correctly and stably perform registration between images.

### Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)^{™}₎, a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.
An image processing apparatus includes: an obtaining unit configured to obtain at least one of first identification information and second identification information; a classification unit configured to classify, using the at least one piece of identification information, the plurality of first bones and the plurality of second bones into a first bone group including a bone that moves in association with a motion of a first part of the subject and a second bone group including a bone that moves in association with a motion of a second part different from the first part; and a registration unit configured to perform first registration between the plurality of first bones and the plurality of second bones classified into the first bone group and second registration between the plurality of first bones and the plurality of second bones classified into the second bone group.

## Claims

1. An image processing apparatus comprising:
an obtaining unit configured to obtain at least one of first identification information that identifies a plurality of first bones depicted in a first image obtained by capturing an image of a subject and second identification information that identifies a plurality of second bones depicted in a second image obtained by capturing an image of the subject;
a classification unit configured to classify, using the at least one piece of identification information, the plurality of first bones and the plurality of second bones into a first bone group including a bone that moves in association with a motion of a first part of the subject and a second bone group including a bone that moves in association with a motion of a second part different from the first part; and
a registration unit configured to perform first registration between the plurality of first bones and the plurality of second bones classified into the first bone group and second registration between the plurality of first bones and the plurality of second bones classified into the second bone group.

2. The apparatus according to claim 1, wherein using the at least one piece of identification information, the classification unit identifies a bone commonly depicted between the first image and the second image, and
a common group in which the commonly depicted bone exists is classified as the first bone group and the second bone group.

3. The apparatus according to claim 1, wherein
using the at least one piece of identification information, the classification unit classifies the plurality of first bones and the plurality of second bones into a third bone group including a bone that moves in association with a motion of a third part different from the first part and the second part, and
the registration unit performs third registration between the plurality of first bones and the plurality of second bones, which are classified into the third bone group.

4. The apparatus according to claim 3, wherein the second part and the third part physically continue to the first part, and the second part and the third part are parts that can move independently of each other.

5. The apparatus according to claim 3, wherein the first part is a trunk of the subject, the second part is one of limbs of the subject, and the third part is one of the limbs of the subject different from the second part.

6. The apparatus according to claim 3, wherein the first bone group includes a rib of the subject, the second bone group includes one of left and right scapula bones of the subject, and the third bone group includes the other of the left and right scapula bones of the subject.

7. The apparatus according to claim 1, wherein
the obtaining unit obtains identification information of a plurality of bones identified from one image of the first image and the second image, and
based on the identification information, the classification unit classifies the plurality of bones identified from the one image, for each part of the subject making a different motion, into a group of bones that move in association with the different motion.

8. The apparatus according to claim 7, wherein the registration unit obtains bone regions from the first image and the second image based on image processing of extracting a region, and
performs registration such that the bone regions match between the first image and the second image.

9. The apparatus according to claim 7, further comprising an association unit configured to associate regions of the plurality of groups of bones classified in one image of the first image and the second image with regions in the other image,
wherein the association unit associates regions of a plurality of groups of bones in the one image classified based on the identification information with regions in the other image.

10. The apparatus according to claim 9, wherein the association unit generates a deformed image by deforming the other image using a displacement field generated in the registration and
associates each of the regions of the plurality of groups of bones in the one image with a corresponding region in the deformed image.

11. The apparatus according to claim 1, wherein the registration unit performs the first registration based on a first displacement field for making an image of a bone region belonging to the first bone group in the first image match an image of a bone region belonging to the first bone group in the second image.

12. The apparatus according to claim 11, wherein the registration unit performs the second registration based on a second displacement field for making an image of a bone region belonging to the second bone group in the first image match an image of a bone region belonging to the second bone group in the second image.

13. The apparatus according to claim 12, further comprising an image generation unit,
wherein the image generation unit
sets a region of the displacement field for the first image,
stores a displacement amount of the first displacement field in a region of the first bone group in the set region,
stores a displacement amount of the second displacement field in a region of the second bone group in the set region, and
generates an integrated displacement field in which a displacement amount of a different displacement field is stored at a different position in the set region.

14. The apparatus according to claim 13, wherein the image generation unit generates a deformed image by, using the integrated displacement field, deforming one medical image of the first image and the second image such that the one medical image matches the other medical image, and
generates a difference image by subtracting pixel information of the deformed image from pixel information of the other medical image.

15. The apparatus according to claim 12, wherein the registration unit associates bone regions identified as identical bone regions between the plurality of first bones and the plurality of second bones based on the first identification information and the second identification information.

16. The apparatus according to claim 15, wherein the registration unit
performs rigid body registration between the bone region in the first image and the bone region in the second image, which are associated, using the first displacement field and the second displacement field as initial displacement fields, and
performs deformation registration capable of setting a degree of freedom of deformation in the bone region between the bone region in the first image and the bone region in the second image, which have undergone the rigid body registration.

17. The apparatus according to claim 14, further comprising a display control unit,
wherein the display control unit performs display control for causing a display unit to display at least one image of the deformed image and the difference image generated by the image generation unit.

18. An image processing apparatus comprising:
an obtaining unit configured to obtain identification information that identifies a plurality of bones depicted in each of a first image obtained by capturing an image of a subject and a second image obtained by capturing an image of the subject;
a classification unit configured to classify, using the identification information, the plurality of bones into a first bone group including a bone that moves in association with a motion of a first part of the subject; and
a registration unit configured to perform first registration between the plurality of bones depicted in the first image and the plurality of bones depicted in the second image, which are classified into the first bone group.

19. The apparatus according to claim 18, wherein
the classification unit classifies, using the identification information, the plurality of bones into a second bone group including a bone that moves in association with a motion of a second part different from the first part, and
the registration unit performs second registration between the plurality of bones depicted in the first image and the plurality of bones depicted in the second image, which are classified into the second bone group.

20. The apparatus according to claim 1, further comprising a determination unit configured to determine whether the second bone group classified in at least one of the first image and the second image includes at least one of a transplant and a bone recognition region in which bone extraction reliability does not satisfy a predetermined threshold.

21. The apparatus according to claim 20, wherein using the identification information obtained by the obtaining unit and information about a result of the determination, the classification unit reclassifies a bone belonging to the second bone group determined to include at least one of the transplant and the bone recognition region in which the bone extraction reliability does not satisfy the predetermined threshold to the first bone group.

22. The apparatus according to claim 1, wherein based on information obtained from the classification unit, the registration unit
obtains first identification information of a first partial bone, which identifies the first partial bone, and first identification information of a second partial bone, which identifies the second partial bone, the first partial bone and the second partial bone being included in the plurality of first bones and classified into the second bone group, and
obtains second identification information of a first partial bone, which identifies the first partial bone, and second identification information of a second partial bone, which identifies the second partial bone, the first partial bone and the second partial bone being included in the plurality of second bones and classified into the second bone group.

23. The apparatus according to claim 22, wherein between the first image and the second image, the registration unit
performs initial registration of the first partial bone based on the first identification information of the first partial bone and the second identification information of the first partial bone, and
performs initial registration of the second partial bone based on the first identification information of the second partial bone and the second identification information of the second partial bone.

24. The apparatus according to claim 23, further comprising a corresponding point group obtaining unit configured to obtain a corresponding point group concerning the second bone group between the first image and the second image based on initial transformation information obtained by performing the initial registration,
wherein the corresponding point group obtaining unit obtains a corresponding point group concerning the first partial bone and a corresponding point group concerning the second partial bone between the first image and the second image.

25. The apparatus according to claim 24, wherein the registration unit
performs deformation processing using the corresponding point group concerning the first partial bone and the corresponding point group concerning the second partial bone as constraint conditions, thereby obtaining integrated transformation information in which initial transformation information generated by initial registration of the first partial bone and initial transformation information generated by initial registration of the second partial bone are put together, and
performs the second registration based on the integrated transformation information.

26. An image processing method comprising:
obtaining at least one of first identification information that identifies a plurality of first bones depicted in a first image obtained by capturing an image of a subject and second identification information that identifies a plurality of second bones depicted in a second image obtained by capturing an image of the subject;
classifying, using the at least one piece of identification information, the plurality of first bones and the plurality of second bones into a first bone group including a bone that moves in association with a motion of a first part of the subject and a second bone group including a bone that moves in association with a motion of a second part different from the first part; and
performing first registration between the plurality of first bones and the plurality of second bones classified into the first bone group and second registration between the plurality of first bones and the plurality of second bones classified into the second bone group.

27. An image processing method comprising:
obtaining identification information that identifies a plurality of bones depicted in each of a first image obtained by capturing an image of a subject and a second image obtained by capturing an image of the subject;
classifying, using the identification information, the plurality of bones into a first bone group including a bone that moves in association with a motion of a first part of the subject; and
performing first registration between the plurality of bones depicted in the first image and the plurality of bones depicted in the second image, which are classified into the first bone group.

28. A storage medium storing a program configured to cause a computer to execute each step of an image processing method defined in claim 27.
